# EUROPEAN PATENT APPLICATION

(11) **EP 3 715 848 A1**
(43) Date of publication of application: **30.09.2020**
(21) Application number: 19305374.1
(22) Date of filing: 25.03.2019
(51) Int. Cl.: G01N 33/574

(54) **SUBSTANCE INTERACTING WITH C TERMINAL FRAGMENT OF THE STIM1 FRACTION LOCALIZED TO THE PLASMA MEMBRANE OF THE CELLS, FOR ITS USE IN THE TREATMENT OF CANCERS, AND IN SCREENING AND DIAGNOSTIC METHODS**

(71) Applicant: Université de Bretagne Occidentale (U.B.O.), 29200 Brest (FR); Centre Hospitalier Régional et Universitaire de Brest, 29609 Brest Cedex (FR); INSTITUT NATIONAL DE LA SANTE ET DE LA RECHERCHE MEDICALE (INSERM), 75013 Paris (FR)
(72) Inventor: MIGNEN, Olivier, 29460 LOGONNA DAOULAS (FR); BUSCAGLIA, Paul, 29200 BREST (FR); HEMON, Patrice, 29800 SAINT DIVY (FR); LE GOUX, Nelig, 22170 PLOUAGAT (FR)
(74) Representative: Cabinet Becker et Associés

(57) **Abstract**

The present invention relates to a substance interacting with C terminal fragment of the STIM1 fraction localized to the plasma membrane of the cells, for its use in the treatment of cancers.

The present invention further relates to a pharmaceutical composition comprising at least one substance interacting with C terminal fragment of the STIM1 fraction localized to the plasma membrane of the cells, and at least one pharmaceutically acceptable excipient.

Another subject-matter of the invention refers to the use of isolated C terminal fragment of the STIM1 fraction localized to the plasma membrane of the cells in a method for screening candidate molecules for treating cancers, especially pancreatic, colon, breast cancer, Burkitt lymphoma or chronic Lymphocytic Leukaemia.

The present invention also relates to a process for predicting the progression and/or monitoring the progression of a cancer, comprising the in vitro detection of the expression of STIM1 in a sample of the tumour of the subject.

SEQUENCE LISTING

<110> UBO
      INSERM
      CHRU de Brest

<120> Substance interacting with C terminal fragment of the STIM1
      fraction localized to the plasma membrane of the cells, for its
      use in the treatment of cancers, and in screening and diagnostic
      methods

<130> BNT225656EP00

<160> 2

<170> PatentIn version 3.5

<210> 1
<211> 685
<212> PRT
<213> Homo sapiens

<400> 1

<210>  2
<211>  452
<212>  PRT
<213>  Homo sapiens

<400>  2

Asn Arg Tyr Ser Lys Glu His Met Lys Lys Met Met Lys Asp Leu Glu
1               5                   10                  15

Gly Leu His Arg Ala Glu Gln Ser Leu His Asp Leu Gln Glu Arg Leu

            20                  25                  30

His Lys Ala Gln Glu Glu His Arg Thr Val Glu Val Glu Lys Val His
        35                  40                  45

Leu Glu Lys Lys Leu Arg Asp Glu Ile Asn Leu Ala Lys Gln Glu Ala
    50                  55                  60

Gln Arg Leu Lys Glu Leu Arg Glu Gly Thr Glu Asn Glu Arg Ser Arg
65                  70                  75                  80

Gln Lys Tyr Ala Glu Glu Glu Leu Glu Gln Val Arg Glu Ala Leu Arg
                85                  90                  95

Lys Ala Glu Lys Glu Leu Glu Ser His Ser Ser Trp Tyr Ala Pro Glu
            100                 105                 110

Ala Leu Gln Lys Trp Leu Gln Leu Thr His Glu Val Glu Val Gln Tyr
        115                 120                 125

Tyr Asn Ile Lys Lys Gln Asn Ala Glu Lys Gln Leu Leu Val Ala Lys
    130                 135                 140

Glu Gly Ala Glu Lys Ile Lys Lys Lys Arg Asn Thr Leu Phe Gly Thr
145                 150                 155                 160

Phe His Val Ala His Ser Ser Ser Leu Asp Asp Val Asp His Lys Ile
                165                 170                 175

Leu Thr Ala Lys Gln Ala Leu Ser Glu Val Thr Ala Ala Leu Arg Glu
            180                 185                 190

Arg Leu His Arg Trp Gln Gln Ile Glu Ile Leu Cys Gly Phe Gln Ile
        195                 200                 205

Val Asn Asn Pro Gly Ile His Ser Leu Val Ala Ala Leu Asn Ile Asp
    210                 215                 220

Pro Ser Trp Met Gly Ser Thr Arg Pro Asn Pro Ala His Phe Ile Met

225                 230                 235                 240

Thr Asp Asp Val Asp Asp Met Asp Glu Glu Ile Val Ser Pro Leu Ser
                245                 250                 255

Met Gln Ser Pro Ser Leu Gln Ser Ser Val Arg Gln Arg Leu Thr Glu
            260                 265                 270

Pro Gln His Gly Leu Gly Ser Gln Arg Asp Leu Thr His Ser Asp Ser
        275                 280                 285

Glu Ser Ser Leu His Met Ser Asp Arg Gln Arg Val Ala Pro Lys Pro
    290                 295                 300

Pro Gln Met Ser Arg Ala Ala Asp Glu Ala Leu Asn Ala Met Thr Ser
305                 310                 315                 320

Asn Gly Ser His Arg Leu Ile Glu Gly Val His Pro Gly Ser Leu Val
                325                 330                 335

Glu Lys Leu Pro Asp Ser Pro Ala Leu Ala Lys Lys Ala Leu Leu Ala
            340                 345                 350

Leu Asn His Gly Leu Asp Lys Ala His Ser Leu Met Glu Leu Ser Pro
        355                 360                 365

Ser Ala Pro Pro Gly Gly Ser Pro His Leu Asp Ser Ser Arg Ser His
    370                 375                 380

Ser Pro Ser Ser Pro Asp Pro Asp Thr Pro Ser Pro Val Gly Asp Ser
385                 390                 395                 400

Arg Ala Leu Gln Ala Ser Arg Asn Thr Arg Ile Pro His Leu Ala Gly
                405                 410                 415

Lys Lys Ala Val Ala Glu Glu Asp Asn Gly Ser Ile Gly Glu Glu Thr
            420                 425                 430

Asp Ser Ser Pro Gly Arg Lys Lys Phe Pro Leu Lys Ile Phe Lys Lys

        435                 440                 445

Pro Leu Lys Lys
    450

## Description

### Technical field

The present invention refers to substances for use in the treatment of cancers, to a method for screening candidate molecules for treating cancers, and to a process for the diagnosis of cancer.

Therefore, the present invention has utility in pharmaceutical field.

In the description below, the references into brackets ([ ]) refer to the listing of references situated at the end of the text.

### Background of the Invention

Pancreatic ductal adenocarcinoma (PDAC) accounts for 90% of all diagnosed pancreatic cancers, and is one of the leading causes of cancer deaths in men and women. In recent years, there has been very little improvement in PDAC patient survival rates. The factors that contribute to the very poor prognosis for patients with PDAC therefore include its rapid progression and invasion, the absence of specific symptoms, and the little impact of the available chemotherapy. The outcome of this cancer is unfortunately almost always fatal despite the available treatments, with a current five-year survival rate of about 6%. Indeed, most patients die within a year after the detection of cancer, especially because about 50% of these patients are diagnosed at invasive metastatic stages of the disease not accessible to surgery.

Despite some progress over the past decade, systemic chemotherapy to treat PDAC has globally limited efficacy and significant toxicity, and PDAC are notoriously resistant to agents molecularly targeted and to immunotherapy. Surgical resection is the reference treatment that is to remove the tumor when still possible; it remains to date the only reliable curative approach. Surgical operation is only considered if the size, location and general condition of the patient allows it. This is the case in 10 to 20% of exocrine pancreatic cancers. When the tumor is unoperable or that it could not be completely removed, different types of chemotherapy, often in combination (gemcitabine, 5-FU, irinotecan, oxaliplatin and nab-paclitaxel), are being considered, and radiotherapy is proposed in case of not operable tumor without distant metastasis.

The favourable therapeutic results remain low in the treatment of PDAC, just like the results of the evaluation of targeted therapies in the treatment of this cancer have been disappointing. This is partly related to the absence of validated biomarkers for the selection of patients eligible for treatment and therefore for the optimal clinical decision. In fact, a major obstacle to effective PDAC treatment is heterogeneity of the disease, which is reflected in various patterns of clinical response to therapy and very heterogeneous clinical evolutions of the disease. It is therefore necessary to identify biomarkers that provide a measure of disease activity and response to treatment for identifying subpopulations of patients likely to benefit to a specific and optimal therapy.

Pancreatic cancer (PC) is thus characterized by extremely high mortality and poor prognosis that are largely attributed to limited effective therapies (in 85% of pancreatic cancer cases), difficulties of early diagnosis and low number of reliable prognostic markers and therapeutic stratification of patients, making this cancer a major public health problem. The PDAC should become the second leading cause of cancer deaths in the United States and Europe by 2030, highlighting the need urging the development of more effective strategies for the control and the detection of pancreatic cancer.

Thus, a need exists of alternative tools for the diagnostic, the stratification, the prognostic and the treatment of pancreatic cancer.

### Description of the invention

Significant work has led the Applicant to develop a new therapeutic target for cancers, especially pancreatic cancer. The Applicant has surprisingly succeeded in demonstrating that STIM1 (Stromal Interacting Molecule 1) protein located at the plasma membrane, and in particular the membrane fraction of the STIM1 protein orientated with its C terminal domain (Cter) located in the extracellular medium, is a specific therapeutic target of cancer, especially pancreatic cancer.

The Applicant identifies for the first time, in the context of the present invention, a calcium influx pathway - the constitutive entry of calcium - regulated by a newly characterized protein, namely the protein STIM1 located at the plasma membrane (mSTIM1). Indeed, the Applicant characterized in pancreatic epithelial cells a new pathway for Ca²⁺ entry that is independent of the release of intracellular calcium stores and is regulated by the STIM1 pool located at the plasma membrane (mSTIM1) of these cells. The Applicant demonstrates the amount of STIM1 expression is correlated to constitutive calcium entry in cancer cell lines.

This Ca²⁺ entry pathway supports the survival of pancreatic epithelial cells, and is involved in their migration.

The Applicant has been able first to demonstrate that the expression of the STIM1 protein in the tumor tissues is correlated with the prognosis of the patients, and that a high expression of STIM1 in these tissues is linked to a decrease in the patient's survival. The measurement of STIM1 expression in cancerous tissues is therefore a new prognostic biomarker. In addition, mSTIM1 protein and the signalling pathways that it regulates have never been proposed as a therapeutic target.

Also, the Applicant has surprisingly demonstrated for the first time that mSTIM1 presents a double topology with an extracellular localization of the N terminal domain of the protein (called N ter out) but also with a C terminal domain orientation (called C ter) of mSTIM1 to the extracellular medium.

Surprisingly, the Applicant demonstrated that mSTIM1 oriented with its extracellular C-terminal domain is present in different cancer cell lines. This dual topology is observed at least in pancreatic cancer cell lines, Burkitt lymphoma cell lines, colon adenocarcinoma cell lines, breast cancer adenocarcinoma cell lines and cell from patients suffering from Chronic Lymphocytic Leukemia.

So, the Applicant proposes mSTIM1 oriented with its extracellular C-terminal domain as a new therapeutic target for pancreatic cancer and the use of substances specifically targeting this part of mSTIM1, especially anti-STIM1 antibodies against C terminal domain of STIM1, as new therapeutic tools. The Applicant demonstrated that treating pancreatic cells with an anti-mSTIM1 antibody targeting the C terminus of STIM1 modulates the constitutive entry of Ca²⁺. Treating the cells with an anti-mSTIM1 antibody targeting the C terminus of STIM1 do not affect store operated calcium entry (SOCEE) in pancreatic cancer cell lines.

Treating pancreatic cancer with anti-mSTIM1 antibody targeting the C terminus of STIM1 induces a decrease in the survival and migration of treated pancreatic cancer cells. Advantageously, the use of such an anti-mSTIM1 antibody according to the invention allows to decrease the survival of targeted cancer cells, in particular by inducing their entry into apoptosis and to reduce their proliferation. Similar observations on cancer cell survival were done for colon and breast cancer cells with effects correlated to the presence of STIM1 Cter out in the plasma membranes of the cells.

Thus, the invention is the first to propose to modulate the activity of mSTIM1 by using a substance targeting the extracellular C ter domain of mSTIM1, for example an antibody directed against the extracellular C ter domain of mSTIM1, in order to modulate cellular responses implied in pancreatic cancer (migration, survival, cell proliferation) and thus bring a new therapeutic solution in the treatment of pancreatic cancer. The substances of the invention, and especially the antibodies, are the first pharmacological modulators specific for the membrane fraction of STIM1 protein with its C terminal end out. It is an alternative to existing treatments, which can advantageously improve their effectiveness while reducing their side effects. The applicant demonstrated an improvement of gemcitabine efficiency for killing pancreatic cancer cells when cells are treated with low dose of gemcitabine along with a low dose of anti-mSTIM1 antibody targeting the C terminus of STIM1.

The use of an anti-STIM1 monoclonal antibody directed against the Cter fraction of STIM1 thus constitutes a novel immunotherapy targeting an alternative signalling pathway to currently targeted signalling pathways in the treatment of cancers, and more particularly pancreatic cancer, Burkitt lymphoma, colon adenocarcinoma, breast cancer adenocarcinoma, Chronic Lymphocytic Leukemia. The development of immunotherapy based on the use of an anti-STIM1 antibody is also a "first in class" therapy to fight against this cancer, and a fortiori against the other types of cancer in which this therapeutic target is present.

Advantageously, such an anti-STIM1 antibody may be used, according to the present invention, alone or in combination with existing chemotherapies, for example gemcitabine.. Advantageously, it may potentiate effects of existing chemotherapies. Advantageously, it may potentiate effects of low doses gemcitabine administered to a patient in need thereof. Also, that immunotherapy can be advantageously proposed in the first line of treatment.

In addition, the Applicant has also demonstrated the interest of STIM1, in the prediction of the evolution of pancreatic cancer.

Accordingly, in a first aspect, the present invention provides a substance interacting with C terminal fragment of the STIM1 fraction localized to the plasma membrane of the cells, for its use in the treatment of cancers.

"STIM1" refers, according to the present invention, to the Stromal Interaction Molecule 1, also referenced in the literature as "GOK". STIM1 is a protein that is encoded in humans by the *STIM1* gene. STIM1 is a multidomain transmembrane protein. The human STIM1 molecule is a protein with sequence ID NO: 1 corresponding to the Uniprot sequence: Q13586 or NCBI: NP_003147.2. This protein is encoded by the sequence corresponding to the NCBI sequence: NM_003156.3 (mRNA transcript). The STIM1 N-terminal region is located in the ER lumen and contains a SAM domain (sterile α motif domain, a protein-protein interaction module) and an EF-hand motif (calcium-binding motif). In the middle of the protein, there is a transmembrane domain, which is followed by a C-terminal region, including a coiled coil, an ERM domain (ezrin-radixin-moesin) and a basic/serine/proline region. The peptide sequence of the C terminal fragment of the STIM1 protein is given in SEQ ID NO: 2 corresponding to amino acids 234 a 685 of the STIM1 protein:

NRYSKEHMKKMMKDLEGLHRAEQSLHDLQERLHKAQEEHRTVEVEKVHLEK
KLRDEINLAKQEAQRLKELREGTENERSRQKYAEEELEQVREALRKAEKELESHSS
WYAPEALQKWLQLTHEVEVQYYNIKKQNAEKQLLVAKEGAEKIKKKRNTLFGTFHV
AHSSSLDDVDHKILTAKQALSEVTAALRERLHRWQQIEILCGFQIVNNPGIHSLVAAL
NIDPSWMGSTRPNPAHFIMTDDVDDMDEEIVSPLSMQSPSLQSSVRQRLTEPQHG
LGSQRDLTHSDSESSLHMSDRQRVAPKPPQMSRAADEALNAMTSNGSHRLIEGVH
PGSLVEKLPDSPALAKKALLALNHGLDKAHSLMELSPSAPPGGSPHLDSSRSHSPS
SPDPDTPSPVGDSRALQASRNTRIPHLAGKKAVAEEDNGSIGEETDSSPGRKKFPL
KIFKKPLKK

STIM1 is mostly localized to the endoplasmic reticulum, and to a much lesser extent to the plasma membrane.

"Fraction of the STIM1 protein localized to the plasma membrane of the cells" or "Fraction of the STIM1 protein localized to the plasma membrane of the cells with its C terminal end out " means, in the sense of the present invention, the fraction of the STIM1 protein localized to the plasma membrane of the cells. Unless otherwise indicated, "STIM1" refers to the total cell fraction, i.e. comprising STIM1 localized in the endoplasmic reticulum membrane and to the plasma membrane, although "mSTIM1" refers to the fraction of STIM1 only located to the plasma membrane.

"C terminal fragment of the STIM1 fraction localized to the plasma membrane of the cells" refers, in the sense of the present invention, to the region between the amino acids 234 and 685 of the STIM1 amino acid sequence SEQ ID NO: 1, i.e. the peptide of sequence SEQ ID NO: 2. The presence of STIM1 at the plasma membrane presenting its C-Terminal end exposed to the extracellular environment is independent of STIM1 glycosylation. The presence of mSTIM1 with its C ter out is regulated by the TGFβ pathway. This pathway is up-regulated in pancreatic cancer and stimulation of pancreatic cancer cells with TGFβ induces an increase in the amount of mSTIM1 with the C ter our orientation.

"Substance" means, in the sense of the present invention, any molecule displaying interaction with C terminal fragment of the STIM1 protein localized to the plasma membrane (mSTIM1), advantageously for inhibiting the constitutive entry of calcium or modulating the presence of mSTIM1 at the plasma membrane in targeted cells to correct all or part of the defects in the cellular functions in which it is involved. The inhibition of the constitutive entry of calcium in targeted cells may be measured or controlled by any means known by the skilled person, for example by studying calcium flows or measuring the variations in intracellular calcium concentration by fluorescence.

The presence of mSTIM1 at the plasma membrane in targeted cells may be measured or controlled by any means known by the skilled person, for example by flow cytometry (Fluorescence activated cell sorting), or Elisa.

The substance may be for example any compound that specifically binds to the C terminal fragment of the mSTIM1 protein. The substance may be of natural or synthetic origin. It may be a protein produced chemically or by any method of bioengineering, such as purification. The substance may notably be identified by applying the method of screening as defined below. Advantageously, the substance is not able to cross the plasma membrane and interacts specifically with the fraction of the STIM1 protein localized to the plasma membrane of the cells without penetrating the cells.

The substance may be for example chosen among an antibody or an isolated binding fragment thereof, a peptide, a protein, a chemical compound and an aptamer. Preferably, it is an isolated antibody or an isolated binding fragment thereof, for example a monoclonal antibody.

The binding of the substance of the invention may be measured by any known techniques, for example by FACS (Fluorescence activated cell sorting), ELISA, or Biacore™ assay.

The phrase "specifically (or selectively) binds to" refers to a binding reaction that is determinative of the presence of the antigen, i.e. the region between amino acid residues 234 and 685 of the STIM1 amino acid sequence SEQ ID NO: 1, in a heterogeneous population of proteins and other biologics. Specific binding between two entities means a binding with an equilibrium constant (K_{A}) (kₒₙ/k_{off}) between 10²M⁻¹ and 5x10¹⁵M⁻¹.

In addition to the equilibrium constant (K_{A}) noted above, the compound of the invention may advantageously also have a dissociation rate constant (K_{D}) (k_{off}/kₒₙ) of 5x10⁻²M or lower, and binds to the antigen as defined above with an affinity that is at least twofold greater than its affinity for binding to a non-specific antigen.

In one embodiment, the compound of the invention may have dissociation constant (K_{d}) of less than 3000 pM as assessed using a method described herein or known to one of skill in the art, e.g., a BIAcore™ assay (Biacore™ International AB, Uppsala, Sweden), ELISA, surface plasmon resonance or FACS.

The term "antibody" as used herein refers to whole antibodies that interact with, e.g., by binding, steric hindrance, stabilizing/destabilizing or spatial distribution, of the C terminal fragment of the STIM1 fraction localized to the plasma membrane of the cells, or to an "antibody fragment", which refers to one or more portions of an antibody that retain(s) the ability to specifically interact with the C terminal fragment of the STIM1 fraction localized to the plasma membrane of the cells. The antibody of the invention may be a naturally occurring antibody, which is a glycoprotein comprising at least two heavy (abbreviated herein as H) chains and two light (L) chains inter-connected by disulfide bonds. Each heavy chain is comprised of a heavy chain variable region (abbreviated herein as VH) and a heavy chain constant region (abbreviated herein as CH). The heavy chain constant region is comprised of three domains, CH1, CH2 and CH3. Each light chain is comprised of a light chain variable region (abbreviated herein as VL) and a light chain constant region (abbreviated herein as CL). The light chain constant region is comprised of one domain, CL. The VH and VL regions can be further subdivided into regions of hypervariability, termed complementarity determining regions (CDR), interspersed with regions that are more conserved, termed framework regions (FR). Each VH and VL is composed of three CDRs and four FRs arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system as effector cells and the first component (Clq) of the classical complement system.

The term "antibody" or "antibody fragment" include for example, monoclonal antibodies, polyclonal antibodies, human antibodies, humanized antibodies, chimeric antibodies, single-chain Fvs (scFv), disulfide-linked Fvs (sdFv), Fab fragments, F(ab') fragments, and anti-idiotypic (anti-Id) antibodies including, e.g., anti-Id antibodies to antibodies of the invention), a monovalent fragment consisting of the VL, VH, CL and CH1 domains; a F(ab)₂ fragment, F(ab₂)', F(ab)₂', scFv, VHH (sdAb fragment, which consists of a VH domain), a Fd fragment consisting of the VH and CHI domains; and an isolated complementarity determining region (CDR). The antibody fragments may be obtained using conventional techniques known to those of skill in the art, and the fragments are screened for utility in the same manner as are intact antibodies.

The framework and/or constant region of the antibody, in the case of an entire antibody, may be from mammals or non-mammals such as human, rodent, camel, canine, feline, shark.

Preferably, the antibody may be a chimeric, a human or a humanized antibody, so that its immunogenicity is reduced in humans, and/or its effectiveness is improved upon therapeutic administration to humans.

The antibodies can be of any isotype for example IgG, IgE, IgM, IgD, IgA and IgY, of any class, for example IgG1, IgG2, for example IgG2b, IgG3, IgG4, IgA1 and IgA2, or of any subclass.

According to a preferred embodiment of the invention, the constant region of each of the light chains of the antibody according to the invention is of κ type. Any allotype is suitable for the implementation of the invention, e.g. Km(1), Km(1, 2), Km(1, 2, 3) or Km(3), but the preferred allotype is Km(3). Preferably, the antibodies of the invention are IgG2b/kappa, especially murine IgG2b/kappa.

The phrase "isolated antibody" as used herein refers to an antibody that has been identified and separated and/or recovered from a component of its natural environment. Contaminant components of its natural environment are materials that would interfere with diagnostic or therapeutic uses for the antibody, and may include enzymes, hormones, and other proteinaceous or non-proteinaceous solutes. Isolated antibody includes the antibody in situ within recombinant cells since at least one component of the antibody's natural environment will not be present. Ordinarily, however, isolated antibody will be prepared by at least one purification step.

For example, the substance may be the anti-STIM1 antibodies the targeting the terminal C (Cter) such as the clone CDN3H4, sc-66173 (Santacruz), the clone HPA012123 (Sigma Aldrich), or the clone HPA011088 (Sigma Aldrich).

The antibody may be manufactured by any method known by the skilled person, for example by culturing a host cell expressing the antibody under conditions that result in production of the antibody, and isolating the antibody of the invention from the host cell or culture medium of the host cell.

The host cell refers to a cell expressing the antibody, by transfection with a nucleic acid molecule or infection with phagemid or a bacteriophage, and the progeny or potential progeny of such a cell. It may be any cell known in the prior art, for example SP2/0, YB2/0, IR983F, Namalwa human myeloma, PERC6, CHO cell, as CHO-DG44, CHO-DUK-B11, CHO-K-1, CHO-Lec10, CHO-Lec1, CHO-Lec13, CHO Pro-5 or CHO/DHFR-, Wil-2, Jurkat, Vero, Molt-4, COS-7, 293-HEK, BHK, K6H6, NS0, SP2/0-Ag 14 or P3X63Ag8.653.

For expression of the nucleic acid, the expression vector(s) may be transfected into the host cell by standard techniques. The various forms of the term "transfection" are intended to encompass a wide variety of techniques commonly used for the introduction of exogenous DNA into a prokaryotic or eukaryotic host cell, such as electroporation, calcium-phosphate precipitation, DEAE-dextran transfection and the like.

Cell culture production, purification and characterization of antibodies can be realized by well-known methods of the prior art. For example, cell can be allow to grow and die (4 to 5 days) before supernatant collection, clarification by low-speed centrifugation and volume reduction by ultra-filtration, for example on Pellicon XL Filter (Millipore). The concentrated culture supernatants can be injected into a HiTrap protein A FF column (GE Healthcare), bound antibodies can be eluted with sodium citrate buffer, and fractions can be neutralized using Tris. Fractions containing the antibodies can be pooled and dialyzed into PBS, and the samples can be sterile-filtered and stored at 4°C. The purified antibodies can be characterized by SDS-PAGE under nonreducing and reducing conditions.

In another embodiment of the invention, the substance may be an aptamer, i.e. nucleic acid molecules having specific binding affinity to non-nucleic acid or nucleic acid molecules through interactions other than classic Watson-Crick base pairing Aptamers may be for example RNA or DNA aptamers. Aptamers are known to be able to substitute for monoclonal antibodies in various applications.

"Cancer" means, in the sense of the present invention, any cancer having a STIM1 fraction localized to the plasma membrane of the cells with at least a part of its C ter end exposed to the external medium. For example, it may be a pancreatic cancer, and more specifically pancreatic ductal adenocarcinoma. It may also be colon cancer (adenocarcinoma), breast cancer (adenocarcinoma), Burkitt lymphoma or Chronic Lymphocytic Leukemia.

It results from the above that the invention also includes a method for treating cancers, comprising administering a medicament comprising a substance interacting with C terminal fragment of the STIM1 fraction localized to the plasma membrane of the cells, to a patient in need thereof.

It also results from the above that the invention additionally includes the use of a substance interacting with C terminal fragment of the STIM1 fraction localized to the plasma membrane of the cells, for the manufacture of a medicament for the treatment of cancers.

A method of treating a cancer, comprising administering a substance interacting with C terminal fragment of the STIM1 fraction localized to the plasma membrane of the cells in a subject in need thereof, is also described.

In a second aspect, the present invention provides a pharmaceutical composition comprising at least one substance interacting with C terminal fragment of the STIM1 fraction localized to the plasma membrane of the cells, and at least one pharmaceutically acceptable excipient.

Such a composition may comprise any excipients and/or additives that facilitate the formulation of the substance in preparations that may be used pharmaceutically. The expression "pharmaceutically acceptable" encompasses any vehicle that does not interfere negatively with the efficacy of the substance for treating cancer, and that is not toxic to the host to whom or to which it is administered. In particular, suitable pharmaceutically acceptable vehicles for a composition according to the invention are vehicles that are suitable in particular for systemic application. Suitable pharmaceutically acceptable vehicles are well known in the prior art and are described for example in Remington Pharmaceutical Sciences (Mack Publishing Company, Easton, USA, 1985 ([2])), a standard reference text in this field. It may be for example one or more components selected from sodium citrate, polysorbate 80, sodium chloride, sodium hydroxide, hydrochloric acid, and water for injection.

Advantageously, the composition according to the invention may find application as a medicinal product. Particularly advantageously, the composition of the invention may find application as a medicinal product in the treatment and/or the diagnosis, stratification and prognostic of cancer as mentioned above.

In the pharmaceutical composition of the invention, the above-mentioned substance may be the only active principal for treatment or diagnosis of cancer. Alternatively, the pharmaceutical composition of the invention may comprise in addition any other active ingredient, for example any other active ingredient that potentiates the effect of the substance or is potentiated by the substance as defined above. It may be for example an association of anti-STIM1 targeting the C terminal of STIM1 and gemcitabine, which decreases pancreatic cell survival compared to the use of active ingredient alone, as demonstrated by the applicant in the Examples.

In a third aspect, the present invention provides the use of isolated C terminal fragment of the STIM1 fraction localized to the plasma membrane of the cells in a method for screening candidate molecules for treating cancers, especially pancreatic cancers.

"Method of screening" means, in the sense of the present invention, any method allowing identification of a substance interacting with the membrane fraction of the STIM1 protein, especially the C-terminal fragment, or modulating its membrane expression. Preferably, the fraction is located on the plasma membrane of intact cells, which means that the plasma membrane is non broken and/or non permeabilized, and advantageously does not allow non-permeant molecules to penetrate the cells. It may be any method known by a person skilled in the art, for example biological screening, for example a technique selected from the group comprising immunofluorescence, FRET, BRET, other luminescence or fluorescence complementation assays, Proximity ligation Assays, Western blot, immunoprecipitation, surface plasmon resonance (SPR), flow cytometry, video microscopy, study of calcium flows, enzyme-linked immunosorbent assay (ELISA), and confocal microscopy, or biophysical screening, for example by measuring the variations in intracellular calcium concentration by fluorescence. Advantageously, the method of screening allows identifying substances that interact selectively with the fraction of the STIM1 protein localized with its C terminal out at the plasma membrane of the cells, without penetrating the cell. In other words, the method of screening allows identifying non-penetrating substances that interact selectively with the fraction of the STIM1 protein localized at the plasma membrane of the cells with its C terminal out. The method of screening may be realized *in vitro,* on a sample containing intact cells expressing on their plasma membrane the mSTIM1 protein.

"Candidate molecule" means, in the sense of the present invention, any molecule that interacts with the C terminal fragment of the fraction of the STIM1 protein localized to the plasma membrane of the cells. The interaction may be as defined above in relation with the substance. For example, the interaction may be a modulation of the activity or expression of this protein. Modulation of the activity of the protein may be reflected in a change of the calcium flows such as a change in the constitutive entry of intracellular calcium. The modulation of expression may be an increase or a decrease in expression of the STIM1 protein localized to the plasma membrane relative to a level measured on the same cell or a comparable cell before application of the candidate molecule. The modulation of expression of the STIM1 protein may for example be linked to transcriptional modifications, epigenetic modifications or changes in mechanisms regulating STIM1 protein incorporation or stability at the plasma membrane, such as modification of STIM1 phosphorylation, ubiquitinylation or modulation of the glycosylation process that is necessary for membrane addressing of the STIM1 protein. Advantageously, the selected candidate molecules interact specifically with the fraction of the STIM1 protein localized to the plasma membrane of the cells. As the selected candidate molecules do not cross the plasma membrane, they do not interact with the STIM1 protein localized to the endoplasmic reticulum in the method of screening of the invention.

"Cells" means, in the sense of the present invention, any cell expressing STIM1 at the level of the plasma membrane and with the C terminus end of STIM1 exposed to the extracellular environment. Advantageously, in the screening method of the invention, cells are intact and/or non broken and/or not permeabilized, in order to strictly screen for molecules that modulate the membrane expression of STIM1 and/or that modulate its functionality such a modification of the constitutive entry of extracellular Ca²⁺. Advantageously, screening method of the invention allows selecting molecules that do not penetrate into the cells and that stay at the plasma membrane, due to their specific interaction with the fraction of the STIM1 protein localized to the plasma membrane. In other words, the method of screening allows selecting non permeant molecules, i.e. molecules that do not cross the plasma membrane. The cells may be isolated cells, and may be provided for the method of screening of the invention in the form of a sample. Advantageously, the cells may be cancer cells.

The use of cells, for example isolated intact, expressing on their plasma membrane the STIM1 protein with C terminus out, in a method for *in vitro* screening candidate molecules useful for treating cancers, especially pancreatic cancer, is included in the invention.

The method of screening may comprise the steps of:
(a) Providing a sample containing isolated intact cells expressing on their surface the STIM1 protein localized to the plasma membrane on the cells with its C terminal fragment localized outside the cell.
(b) Treating the sample with a candidate molecule under conditions allowing to test the effect of the candidate molecule on the presence of the fraction of the mSTIM1 protein with its C terminal end out.
(c) Treating the sample with a candidate molecule under conditions allowing to test the effect of the candidate molecule on the functionality or the presence of the fraction of the mSTIM1 with its C terminal end out.
(d) determining the binding between the candidate molecule and the C terminal fragment of the mSTIM1 protein, thereby identifying and selecting a compound that specifically binds, as defined above, to the STIM1 fraction localized to the plasma membrane of the cells with its C terminal fragment out.

In another aspect, the present invention provides a process for predicting the progression and/or monitoring the progression of a cancer, comprising the *in vitro* detection of the expression of STIM1 in a biological sample from the subject. Advantageously, the measurements of STIM1 are made in the total cell fraction STIM1.

The diagnosis of a cancer, for example a pancreatic cancer in a subject, may be realised by measuring STIM1 expression in pancreatic cells. A STIM1 expression in these cells that is reduced compared to STIM1 level in control pancreatic non-cancerous cells means that these cells are cancerous, and thereby allows diagnosing pancreatic cancer in the subject.

By "predicting the progression and/or monitoring the progression" it is meant in the invention that the method allows to predict the likely outcome of a cancer. More particularly, the prognosis method can evaluate the survival rate, said survival rate indicating the percentage of people, in a study, who are alive for a given period of time, usually 5 years, after diagnosis of a cancer. This information allows the practitioner to determine if a medication is appropriated, and in the affirmative, what type of medication is more appropriate for the patient.

The prediction of progression and/or the monitoring of the progression of a cancer, for example a pancreatic cancer in a subject, may be realised by measuring STIM1 expression in pancreatic cancer cells. An increase of STIM1 expression in tumor tissues over time in the subject is associated with a decreased overall survival in pancreatic cancer, i.e. a bad prognosis of the cancer.

The change in expression may be a increased expression of STIM1 within the tumor, thereby allowing a bad prognosis or progression of cancer. "Increased expression" means, in the sense of the invention, an expression with a grade of more than 2 using our IHC expression scale.

Generally, a good prognosis, in the sense of the invention, is associated with median survival higher than 3 years, whereas a bad prognosis is associated with a median survival lower than 3 years.

For the measurement of STIM1 expression, STIM1 detection may be realized with any compound able to recognize specifically any part of STIM1, or preferably the C terminal fragment of the STIM1. It may be for example an antibody or a binding-fragment thereof, a protein, a peptide, a chemical compound or an aptamer. Preferably, detection is made with an antibody directed against the C terminal fragment of the STIM1 fraction located at the cell plasma membrane, for example anti-STIM1 antibodies (STIM1: clone CDN3H4, sc-66173, Santacruz) targeting the terminal C fragment of mSTIM1.

This invention is further illustrated by the following examples with regard to the annexed drawings that should not be construed as limiting.

### Brief description of the figures

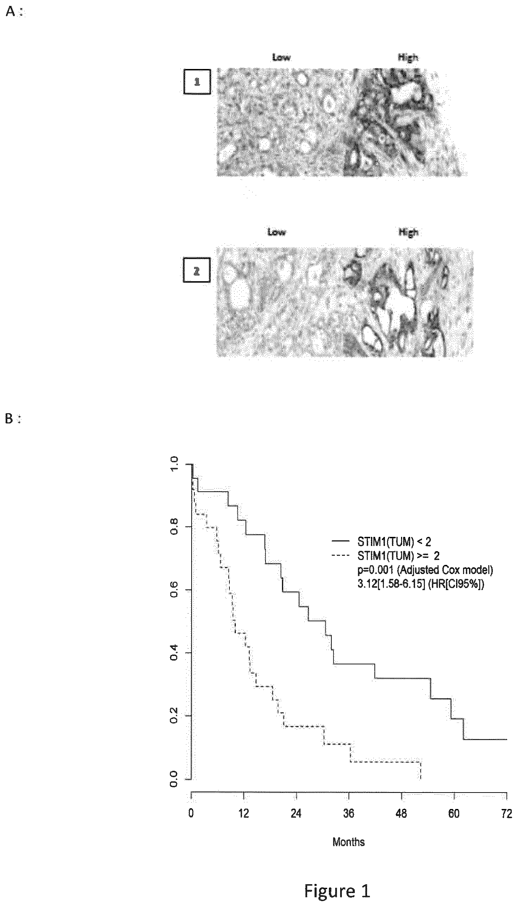

- Figure 1: shows that STIM1 expression is reduced in pancreatic cancer cells but its increase is associated with a decreased overall survival in pancreatic cancer. A: Expression of STIM1 protein in pancreatic tissues is detected by immunochemistry and ranked in two grades (low and high STIM1 expression). Representative images of STIM1staining in pancreatic primary tumors obtained from human pancreas cancer resections (STIM1 antibodies from Sigma-Aldrich clones HPA011088 (1) and N6072 (2)). B: representative Kaplan-Meieroverall survival curves based on the expression of STIM1 in tumor tissues. Patients are split in two groups: high (STIM1(TUM≥2, high grade, dashed line) and low (STIM1(TUM<2, low grade, full line) STIM1 expression (n=48). Data are expressed as t-test of student and Cox analysis, *P<0.05, **P<0.01 and ***P<0.001.

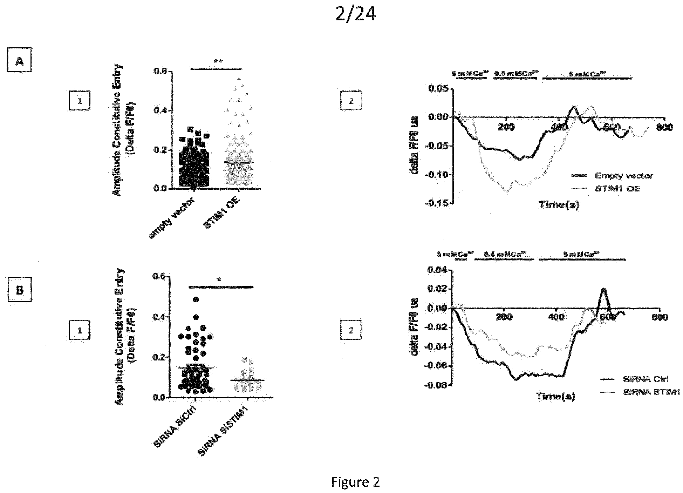

- Figure 2: shows that STIM1 is implicated in the constitutive calcium entry of Panc-1-Wt cells. Panels A and B: Constitutive entry is evaluated by recording intracellular Ca²⁺ concentration variations consequent to changes in extracellular Ca²⁺ concentrations. Constitutive Ca²⁺ entry is regulated by STIM1 expression as observed by the amplitude of this entry in Panc-1-Wt cells overexpressing (STIM1 OE; n=150; triangles, Panel A1) or under-expressing (SiRNA targeting STIM1; n>40; grey squares, Panel B1) compared to values obtained in cells transfected with an empty vector (squares) or an untargeted SiRNA (SiRNA Ctrl, circles). Representatives recording of constitutive Ca²⁺ entry measurements are presented for the different conditions (Panels A2: black line=empty vector, grey line=STIM1OE and B2: black line=siRNA Ctrl, grey line=siRNA STIM1). Data are expressed as mean +/- SEM of n observations and analyzed with a non parametric Mann Withney test, *P<0.05, **P<0.01 and ***P<0.001.

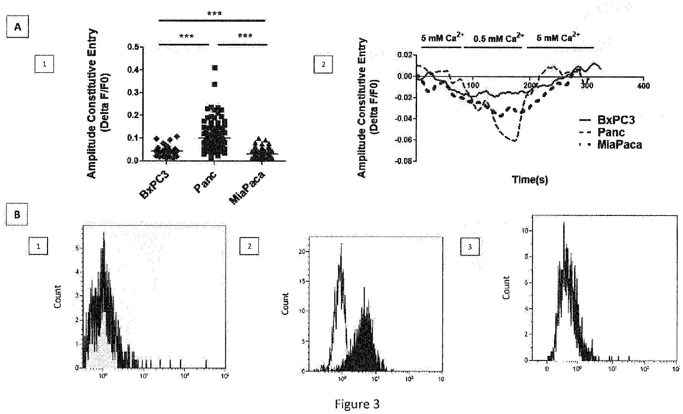

- Figure 3: shows that mSTIM1 expression is correlated to constitutive calcium entry in different pancreatic cancer cell lines BxPC3, Panc-1-Wt and MiaPaca. Panel A1 : Constitutive entry is evaluated by recording intracellular Ca²⁺ concentration variations consequent to changes in extracellular Ca2+ concentrations. Histograms of constitutive Ca2+ entry amplitudes measured in BxPC3 (diamonds), Panc-1-Wt (squares) and MiaPaca (trianlges) cell lines are presented in Panel A1. Representatives recordings of constitutive Ca2+ entry measurements are presented for the different Pancreatic cell lines in Panel A2 (BxPC3: black line, MiaPaca: grey line, Panc: dark grey). Data values are plotted as individual experimental points and the mean +/- SEM of n observations is also reported. Data are analyzed by non parametric Mann Withney analysis, ***P<0.001. Panel B: Amount of mSTIM1 observed in the different pancreatic cell lines (Panc-1-Wt >MiaPaca>BxPC3) is proportional to the amplitude of constitutive Ca²⁺ entry measured in these cell lines. Representative overlays of mSTIM1 level detected by flow cytometry in BxPC3 (Panel B1) Panc-1-Wt (Panel B2) and MiaPaca (Panel B3) cells. STIM1 labeling in non permeabilized cells was realized with an anti-STIM1 antibody clone GOK (targeting the STIM1 N-terminal part) coupled to PE (dark grey) and compared to the labeling observed with an isotype (light grey).

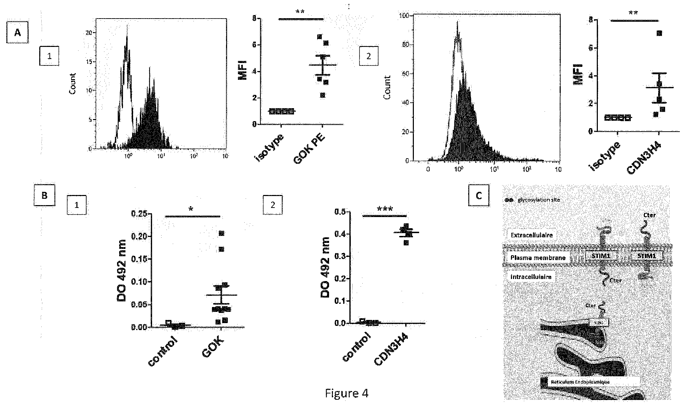

- Figure 4: shows that the fraction of STIM1 located at the plasma membrane (mSTIM1) has a dual topology in pancreatic cells Panel A: mSTIM1 dual topology on Panc-1-Wt cells plasma membrane was identified by flow cytometry. Line graphs shows representative overlays of mSTIM1 expression (dark grey) detected in non permeabilized cells with a STIM1 antibody targeting the STIM1 N-terminal region (GOK Ab coupled to PE - Panel A1) or with a STIM1 antibody targeting the STIM1 C-terminal region of STIM1 (CDN3H4 clone coupled to PE - Panel A2). Light gray: isotype control. Histograms represent the mean Mean Fluorescence Intensity values (MFI) ± SEM of mSTIM1 expressing cells (n=6). Panel B: mSTIM1 dual topology on Panc-1-Wt cells plasma membrane was confirmed by an Elisa approach. Histogram display values of optical densities (OD) ± SEM measured in intact Panc-1-Wt cells using antibodies targeting either the N-terminal region (GOK Ab, n=12 - Panel B1) or the C-terminal region (CDN3H4 clone, n=4 - Panel B2) of STIM1. Panel C: schematic representation of mSTIM double orientation. Data are expressed as mean +/- SEM of n observations and analysed by non parametric Mann Withney analysis, *P<0.05, **P<0.01 and ***P<0.001.

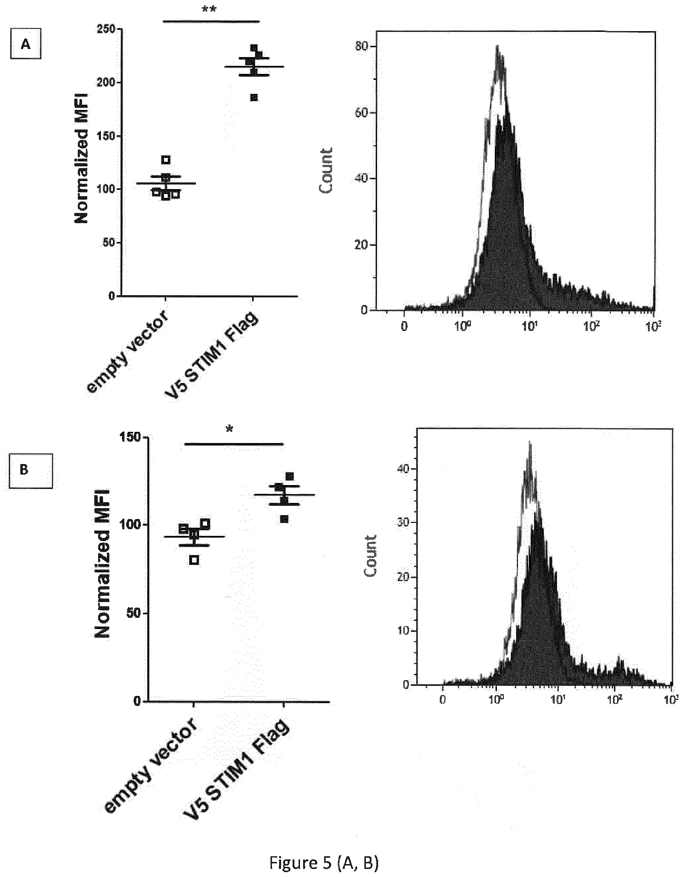

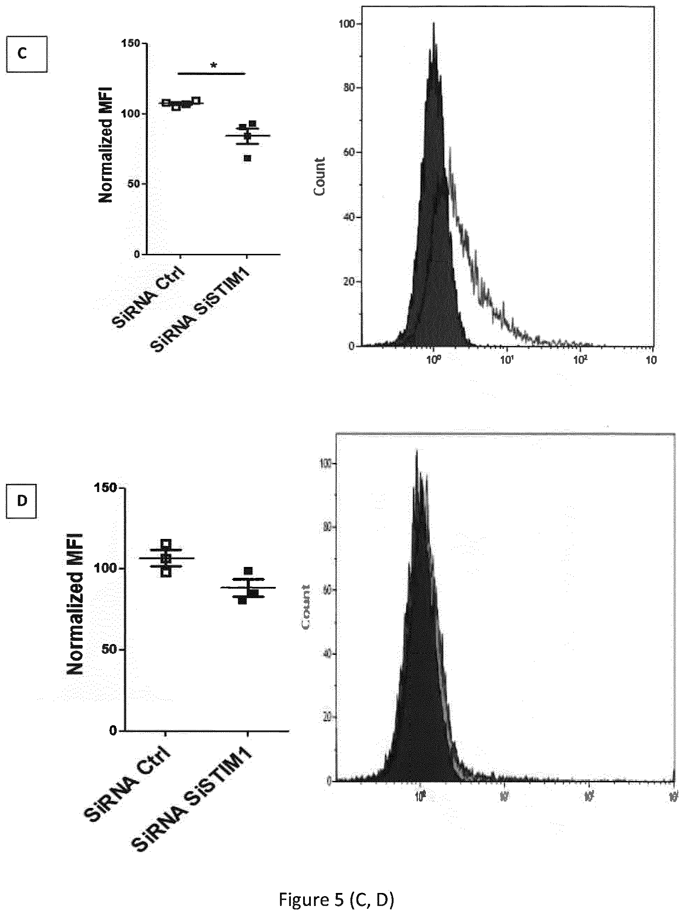

- Figure 5: shows that changing total STIM1 expression confirmed mSTIM1 dual topology in Panc-1-Wt cell line. STIM1 mAb reactivity in PANC cells as assessed by direct immunofluorescence staining with flow cytometry analysis in non permeabilized Panc-1-Wt cells transfected with a STIM1 expression vector (V5STIM1Flag: Panel A and B) or a SiRNA targeting STIM1 (SiSTIM1: Panel C and D). Intact cells are labeled with a STIM1 antibody targeting the STIM1 N-terminal region (GOK Ab coupled to PE - Panel A and C) or with a STIM1 antibody targeting the C-terminal region of STIM1 (CDN3H4 clone coupled to PE - Panel B and D). Representative plots of fluorescence of Panc-1-Wt cells stained with STIM1 antibody (light and mid grey) or isotype-matched control (dark grey) mAbs are shown on a four-decade log scale for each condition. Histograms of Mean Fluorescence intensities (MFI) values are provided for each experimental conditions. MFI values are normalized to control conditions (transfection with an empty vector or a non targeting SIRNA (SiRNA Ctrl). Data are expressed as mean +/- SEM of n observations and analyzed by non parametric Mann Withney analysis, *P<0.05 and **P<0.01.

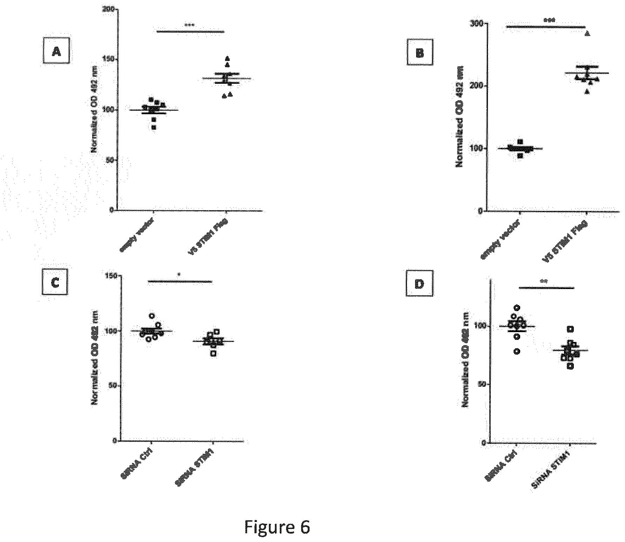

- Figure 6 represents the analysis of mSTIM1 topology by Elisa in pancreatic cancer cell expressing various amount of STIM1. Quantification by Elisa of the amount of STIM1 located at the plasma membrane (Panels A and C) or of total STIM1 expression (Panels B and D) using an antibody targeting a C-Terminal STIM1 epitope (CDN3H4 clone). Histogram display values of optical densities (OD) ± SEM measured in Panc-1-Wt cells transfected with V5 STIM1 Flag normalized and compared to empty vector (Panel A and B; n=8) or transfected with an SiRNA targeting STIM1 (Panel C and D; n>6). Mean values ± SEM are reported for each experimental condition. Data are normalized to control values (transfection with an empty vector or a non targeting SIRNA) and analyzed by non parametric Mann Withney analysis, *P<0.05, **P<0.01 and ***P<0.001.

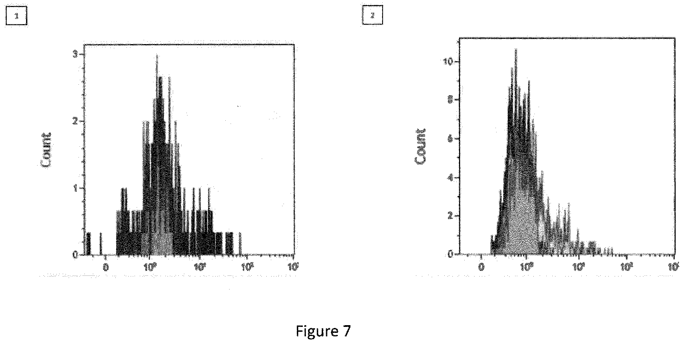

- Figure 7 shows that STIM1 located at the plasma membrane has a dual topology in different pancreatic cancer cells. Representative Flow cytometry plots of plasma membrane STIM1 expression in BxPC3 (Panel 1) and MiaPaca (Panel 2) cell lines. Intact cells were labelled with a antibody recognizing an epitope located in the C-terminus of STIM1 (CDN3H4 clone coupled to PE in light grey PE or isotype in dark grey).

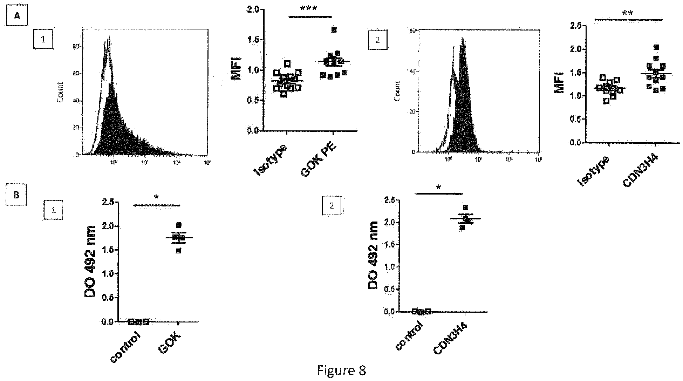

- Figure 8: shows that STIM1 located at the plasma membrane (mSTIM1) of B lymphocyte cell lines has a dual topology. Panel A: Intact JOK Cells were labeled with anti-STIM1 antibodies recognizing the STIM1 N-terminal region (GOK Ab coupled to PE - Panel A1) or the STIM1 Cterminal region (CDN3H4 clone coupled to PE - Panel A2). Representative cytometry plots of mSTIM1 are representative of 11 experiments with the isotype and anti-STIM1 antibody labeling in dark grey and light grey respectively. Values of Mean Fluorescence intensity values (MFI) (n=11) of STIM1 expression are presented in histograms along with the mean value ± SEM . Panel B: : Detection by Elisa of mSTIM1 in PLP cells incubated with STIM1 clone GOK (Panel B1) or clone CDN3H4 (Panel B2) antibodies or with secondary antibody only (control). Mean +/- SEM of 4 observations is reported along with single values . Data are analysed by non parametric Mann Withney analysis, **P<0.01 and ***P<0.001.

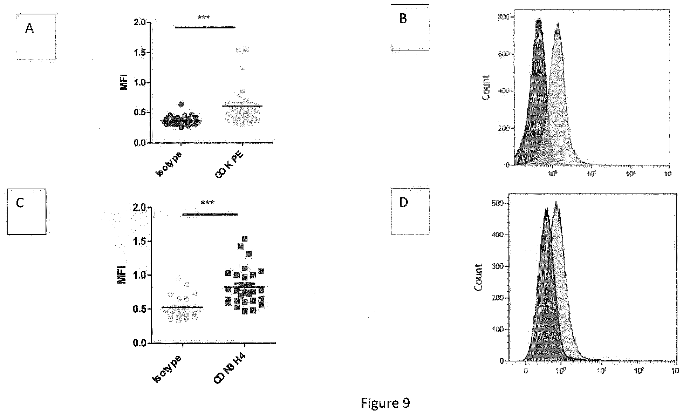

- Figure 9: shows that STIM1 has a dual topology when located at the plasma membrane of B lymphocytes from Chronic Lymphocytic Patients. mSTIM1 detection in intact B cells was tested by flow cytometry immediately after isolation from the peripheral blood . Representative cytometry plots illustrating the labeling in B cells of STIM1 with a STIM1 antibody targeting either the STIM1 N-terminal region (GOK Ab coupled to PE - Panel A) or with a STIM1 antibody targeting the STIM1 C-terminal region of STIM1 (CDN3H4 clone coupled to PE - Panel B)- Labeling with Isotype control is in dark grey. Histogram of Mean Fluorescence Intensity (MFI) values are presented. Mean +/- SEM of n (at least 50) observations is presented on each histogram and data are analyzed by non parametric Mann Withney analysis, ***P<0.001.

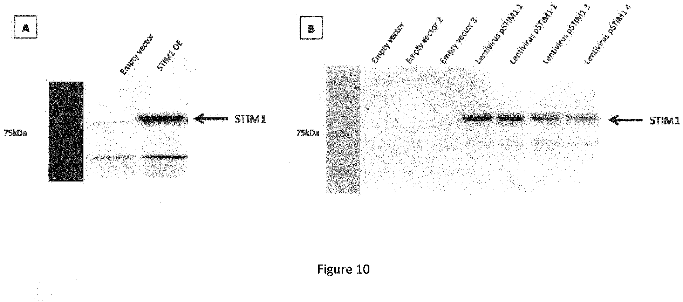

- Figure 10 represents the validation by Western Blot of the selectivity of the antibody targeting the C terminus of STIM1 clone CDN3H4. Expression of STIM1 was revealed by Western Blot using an antibody targeting a C-Terminal STIM1 epitope (CDN3H4 clone) in Panc-1-Wt cells (Panel A) or HEK (Panel B) over-expressing STIM1. Panc-1-Wt cells were transfected with a expression vector containing STIM1 (STIM1 OE) or empty vector whereas HEK cells were transduced with a lentivirus containing STIM1 (pSTIM1) or an empty vector.

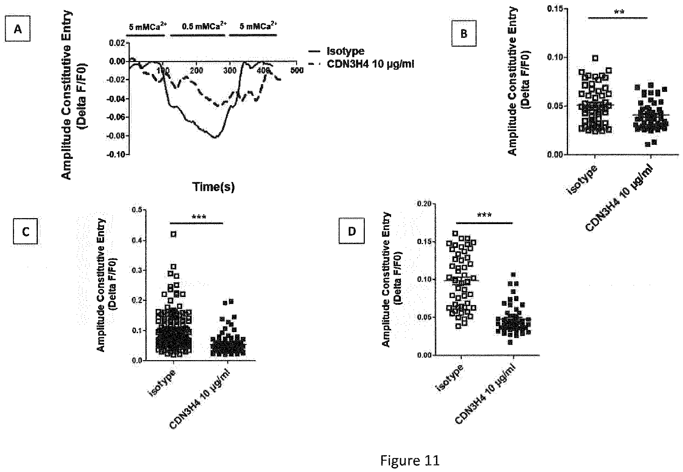

- Figure 11 shows that constitutive Ca²⁺ entry of pancreatic cancer cells is blocked by an anti-STIM1 antibody targeting the STIM1 C-Terminal region. A: Constitutive Ca²⁺ entry is measured by changing external Ca²⁺ concentration in pancreatic cancer cells incubated with an anti-STIM1 antibody targeting the C terminal domain of STIM1 (CDN3H4 clone light grey) or an isotype (dark grey). Representative recording of this measurement in Panc-1-Wt cells is presented in Panel A. Histograms display single values of Ca²⁺ constitutive entry amplitudes along with the with mean amplitude value +/- SEM of the n observations (n>70 cells) in BxPC3 (Panel B), Panc-1-Wt (Panel C) and MiaPaca (Panel D) cell lines. Data are analyzed by with non parametric Mann Withney analysis, **P<0.01 and ***P<0.001.

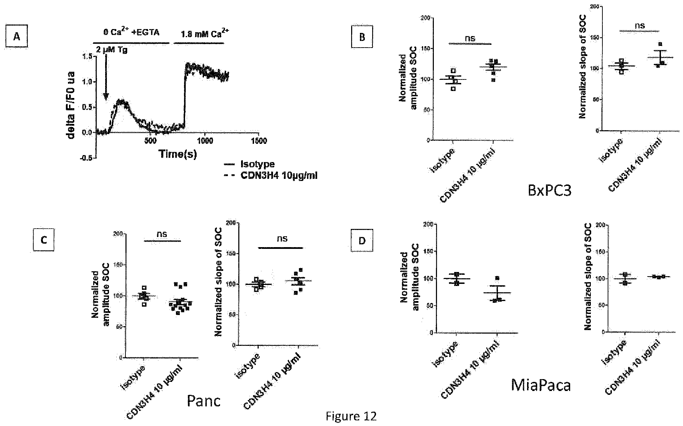

- Figure 12: shows that Store Operated Ca²⁺ entry of pancreatic cancer cells is not modulated by an anti-STIM1 antibody targeting the STIM1 C-Terminal region. A: SOCE is recorded after endoplasmic reticulum Ca²⁺ store depletion with Thapsigargin (2 µM) in 0 mM external Ca²⁺ and re-addition of 1.8 mM extracellular Ca²⁺. Pancreatic cells were incubated with an isotype or with the anti-STIM1 antibody targeting the C terminal end of STIM1 (CDN3H4) as illustrated in Panel A for Panc-1-Wt cells. Histograms display the individual values of SOCE amplitude and speed (slope) and mean values +/- SEM of n observations (n>3) in BxPC3 (Panel B), Panc-1-Wt (Panel C) and MiaPaca (Panel D) cell lines. Data are analyzed by non parametric Mann Withney analysis, ns: non significant.

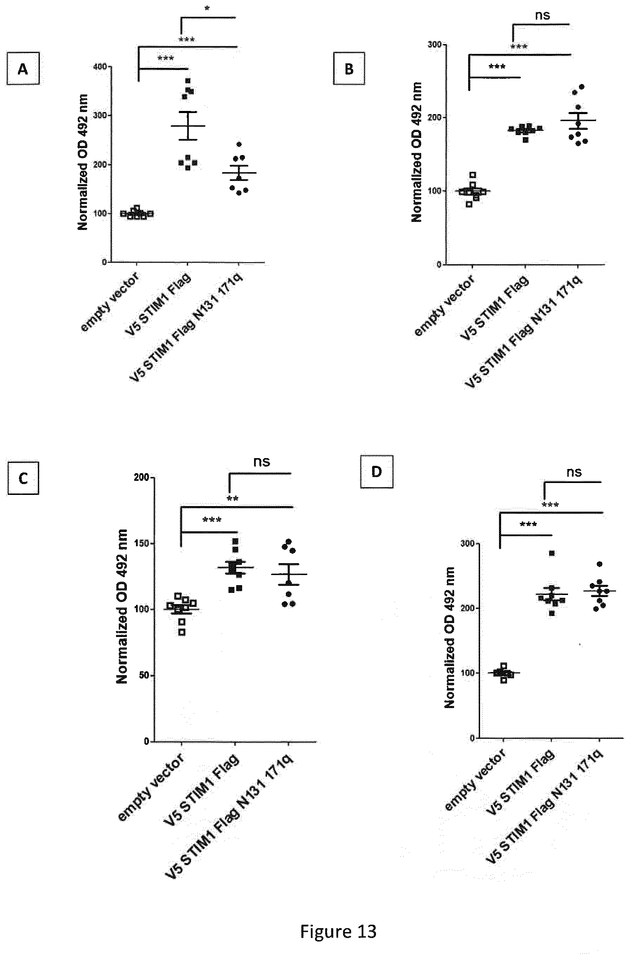

- Figure 13: shows that the presence of STIM1 at the plasma membrane presenting its C-Terminal end exposed to the extracellular environment is independent of STIM1 glycosylation status in pancreatic cells. The amount of mSTIM1 at the plasma membrane is evaluated on Panc-1-Wt cell lines by Elisa using antibodies targeting the N-Terminal (GOK clone, Panel A and B) or C-terminal (CDN3H4 clone, Panel C and D) domains of STIM1. Histogram represent individual values of optical density (OD) and their mean +/- SEM of n observations (n >7) for each experimental conditions. Amount of mSTIM1 (Panel A and C) and total STIM1 (B and D) were evaluated in cells transfected with expression vectors containing wild type STIM1 (V5 STIM1F lag), a STIM1 mutated at the glycosylation sites (V5 STIM1 Flag N131 171) or an empty vector. Values are normalized to the OD measured in cells transfected with the empty vector absorbance. Data are analyzed by non parametric Mann Withney analysis, **P<0.01 and ***P<0.001.

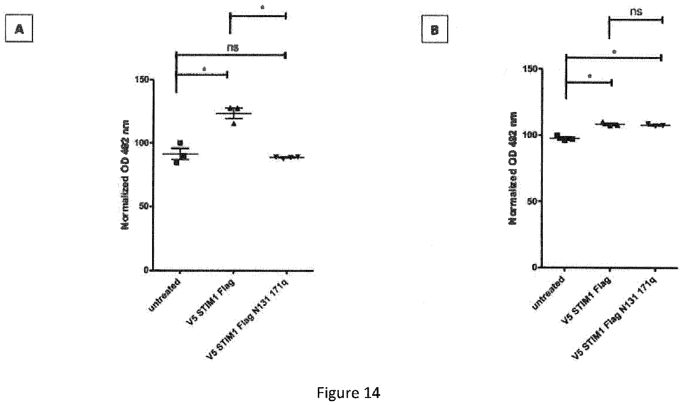

- Figure 14: shows that the localization of STIM1 at the plasma membrane with its C-Terminal outside the cell is independent of STIM1 glycosylation status in pancreatic cells. Amount of mSTIM1 was evaluated by Elisa in MiaPaca cells using an antibody targeting the N terminal (GOK clone Panel A) or the C-terminal (CDN3H4 clone, Panel B) domains of STIM1. Histogram represent singles values of optical densities and the mean +/- SEM of n observations (n >4) detected in MiaPaca cells transfected with expression vectors containing wild type STIM1 (V5STIM1Flag), a STIM1 mutated at the glycosylation sites (V5STIM1Flag N131 171) or an empty vector. Values are normalized to the OD measured in cells transfected with the empty vector absorbance . Data are analyzed with non parametric Mann Withney analysis, *P<0.05, **P<0.01 and ***P<0.001.

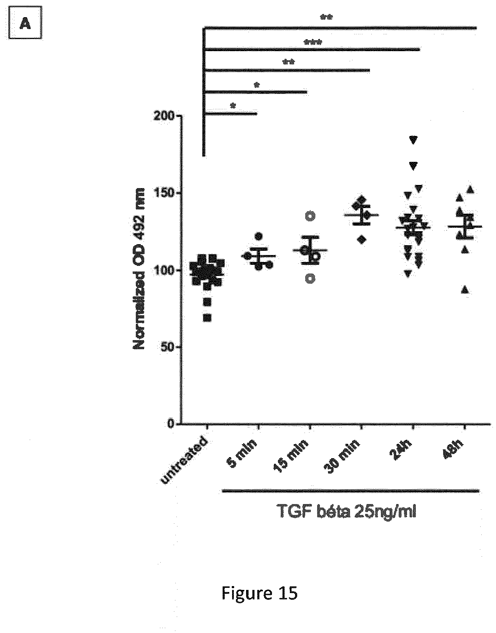

- Figure 15: shows the regulation of the presence of STIM1 at the plasma membrane with its C-Terminal out by the TGFβ1 signaling pathway in pancreatic cancer cells. An ELISA experimental approach was used to detect mSTIM 1 (intact cells) and total STIM1 expression (permeabilized cells) using an antibody targeting the C-terminal domain of STIM1 in Panc-1-Wt cells treated with TGFβ1 for different periods of time. Histogram shows single values of optical densities (OD) and their mean +/-SEM of the n observations for the different experimental conditions. Data are analyzed by non parametric Mann Withney analysis, *P<0.05, **P<0.01 and ***P<0.001.

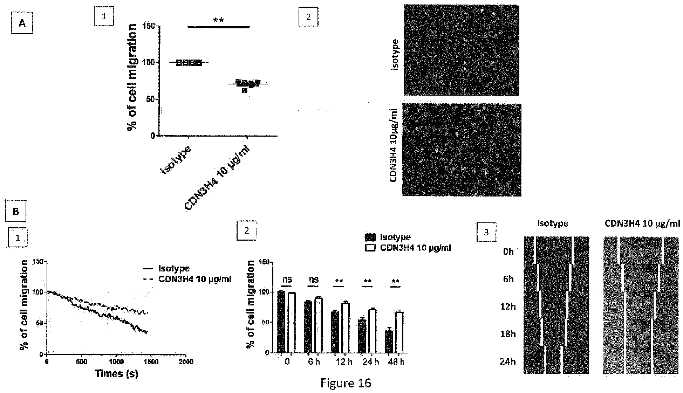

- Figure 16: shows the *in vitro* functional effects of an antibody targeting the C terminal domain of STIM1 in pancreatic cancer cells. Migration of Panc-1-Wt cells is evaluated for 48 hours using a transwell migration assay with boyden chambers (Panel A, n=8) or by the analysis of wound healing (Panel B, n=12). Cells were incubated all along the experiences with 10 µg/ml of an anti-STIM1 antibody targeting the C terminal domain of STIM1 (CDN3H4 clone). Histograms (Panel A1 and B2) show single values of the % of migration as well as the mean +/- SEM of n experiments. A representative evolution over time of wound healing is presented in panel B1. Representative experiments in Boyden chambers or in wound healing test are illustrated in panels A2 and B3. Data are analyzed by non parametric Mann Withney analysis, *P<0.05, **P<0.01 and ***P<0.001.

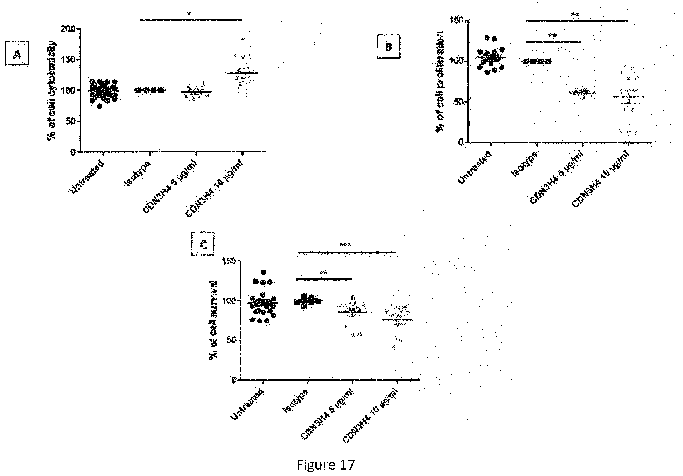

- Figure 17 illustrates the *In vitro* effects on pancreatic cancer cell survival of an antibody targeting the C terminal domain of STIM1. In each experimental approach, cells were treated for 48 hours with 2 different concentrations (5 and 10 µg/ml) of the antibody (CDN3H4 clone) targeting the C terminal domain of STIM1 or with a control isotype. Panel A: Evaluation of the cytotoxic effect (Cell Tox green assay, Promega) on Panc-1-Wt cells of the antibody (clone CDN3H4, n=16). Panel B: Evaluation of cell proliferation (Cell Titer proliferation assay, Promega) of cells treated with the antibody (CDN3H4 clone; n=18). Panel C: Analysis of Panc-1-Wt cell survival (CCK-8 kit, Sigma) when cells were treated with different concentrations of the antibody (CDN3H4 clone, n=12). Histograms represent for each experimental conditions individual values and the mean +/- SEM of n observations. Data were normalized to the mean values obtained for cells treated with the isotype. Data are analyzed by non parametric Mann Withney analysis, *P<0.05, **P<0.01 and ***P<0.001.

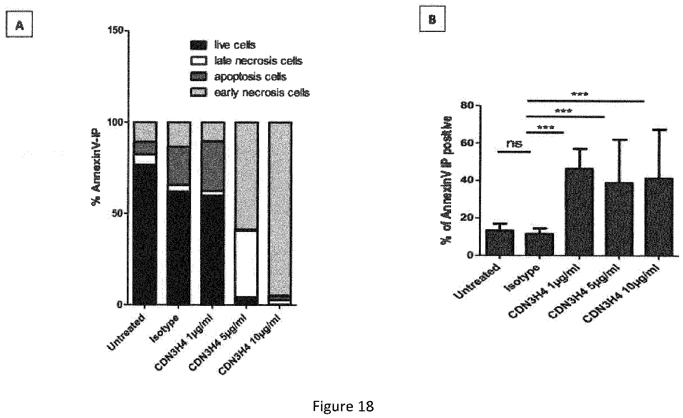

- Figure 18 shows the *in vitro* effects on cell death of pancreatic cancer cells off an antibody targeting the C terminal domain of STIM1. Panc-1-Wt cells are treated with an antibody targeting the C terminal domain of STIM1 (CDN3H4 clone) for 48 hours of treatment with 3 different concentrations of the anti-STIM1 antibody (1, 5 and 10 µg/ml) or with an isotype. Cell death was analysis by flow cytometry by Anexin/Propidium Iodide labeling. Percentage of live cells (black), necrotic cells (late necrosis cells=white, early necrosis cells=light grey) or apoptotic cells (dark grey) submitted to antibody treatment are normalized to the isotype treatment conditions (Panel A). Histogram from panel B present the amount of apoptotic cells (anexin/PI positive cells) detected in each experimental conditions. Data are expressed as mean +/- SEM of n observations (n=3). Data are analyzed with non parametric Mann Withney analysis, *P<0.05, **P<0.01 and ***P<0.001.

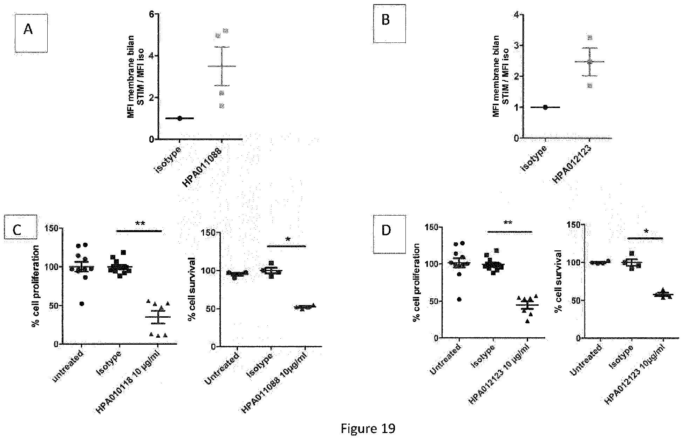

- Figure 19 demonstrates that targeting C-Terminal out mSTIM1 to modulate pancreatic cancer cells proliferation using different STIM1 antibody targeting the C terminal end of STIM1 . Panel A and B: mSTIM1 C-Terminal out detected on Panc-1-Wt cells plasma membrane by flow cytometry using anti-STIM1 C-Terminal antibody (HPA011088) in panel A and anti-STIM1 C-Terminal antibody (HAP012123) in Panel B. MFI of STIM1 labeling is normalize to isotype labeling (n>3). Panel C and D: In each experimental approach, cells were treated for 48 hours with 10 µg/ml of the anti-STIM1 antibody (HPA011088 in panel C and HPA012123 in panel D) targeting the C terminal domain of STIM1 or with a control isotype. Cell proliferation (using the Cell Titer assay Promega) and cell survival (using CCK8 assay Sigma) were evaluated in cells treated with the antibody (HPA011088 or HPA012123; n>4). Histograms represent for each experimental conditions individual values and the mean +/- SEM of n observations. Data were normalized to the mean values obtained for cells treated with the isotype. Data are analyzed by non parametric Mann Withney analysis, *P<0.05, **P<0.01 and ***P<0.001.

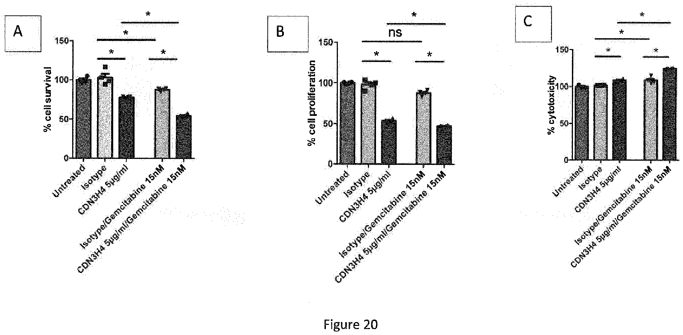

- Figure 20 shows the benefits *In vitro* of associating the chemotherapeutic agent Gemcitabine with an antibody targeting the C terminal domain of STIM1 to reduce pancreatic cancer cell survival. In each experimental approach, cells were treated for 48 hours with 5 µg/ml of the antibody targeting the C terminal domain of STIM1 (CDN3H4 clone) alone or associated with Gemcitabine (15nM). Cells were treated with a control isotype in control conditions. **Panel A:** Evaluation in Panc-1-Wt of the effects of the treatments on cell survival (CCK8 kit, **Panel A**)**,** on cell proliferation (Cell Titer assay, **Panel B**) on cell cytotoxicity (Cell Tox green assay, **Panel C**)**.** Histograms (n=4 in each condition) represent for each experimental conditions individual values and the mean +/- SEM of n observations. Data were normalized to the mean values obtained for cells treated with the isotype. Data are analyzed by non parametric Mann Withney analysis, *P<0.05, **P<0.01 and ***P<0.001.

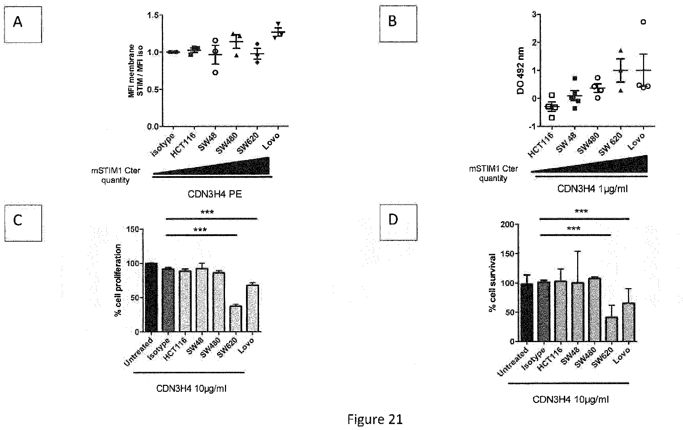

- Figure 21 illustrates that in colorectal adenocarcinoma cells the expression gradient of mSTIM1 with its C-Terminal out is correlated with the effects on cell survival of anti-STIM1 mAbs targeting the C terminus of STIM1. Panel A-B: Amount of mSTIM1 C-Terminal out in the plasma membrane of colorectal adenocarcinoma cells was obtained by flow cytometry experiments (panel A) or by an Elisa approach (panel B). Histograms represents the mean Mean Fluorescence Intensity values (MFI) ± SEM of mSTIM1 expressing cells (n>3) compared to isotype (panel A) or mean values of optical densities (OD) ± SEM measured in intact cells using antibodies targeting either the C-terminal region (CDN3H4 clone, N>3) of STIM1 (panel B). Panel C and D: Effects on cell proliferation (panel C) and cell survival (panel D) of a 48 hours treatment with 10 µg/ml of an antibody (CDN3H4 clone) targeting the C terminal domain of STIM1 or with a control isotype. Evaluation in panel C of cell proliferation (Cell Titer assay) and of cell survival (CCK8 kit) in panel D when cells were treated with the anti-STIM1 antibody clone CDN3H4 (n=12 in each conditions). Histograms represent for each experimental conditions individual values and the mean +/- SEM of n observations. Data were normalized to the mean values obtained for cells treated with the isotype. Data are analyzed by non parametric Mann Withney analysis, *P<0.05, **P<0.01 and ***P<0.001.

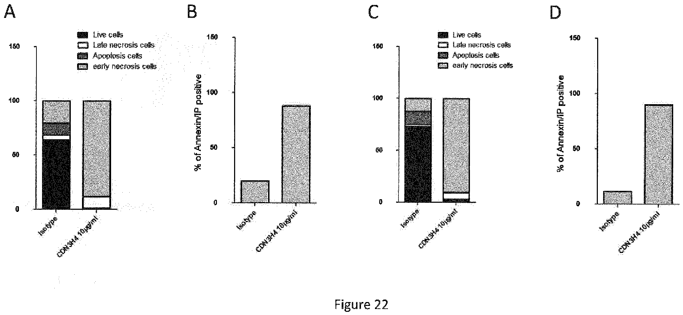

- Figure 22 shows the *in vitro* effects on colorectal adenocarcinoma (SW620 and Lovo) cell death of an antibody targeting the C terminal domain of STIM1 (CDN3H4 clone). LOVO cells (panels A-B) and SW-620 cells (panels C-D) are treated with 10 µg/ml of CDN3H4 antibody for 48 hours or with an isotype. Cell death was analysis by flow cytometry using an Anexin/Propidium Iodide labeling protocol. Percentage of live cells, necrotic cells or apoptotic cells submitted to the antibody treatment are normalized to the isotype treatment condition (Panels A-C). Histogram from panels B-D present the amount of necrotic cells (anexin/PI positive cells) detected in each experimental conditions. Data are expressed as mean +/- SEM of n observations (n=1).

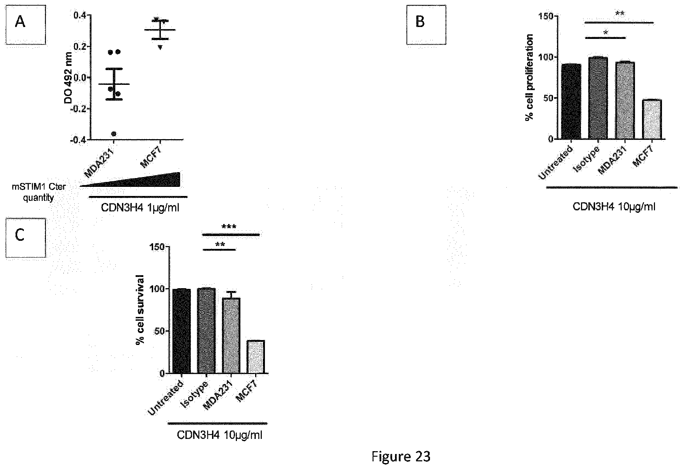

- Figure 23 outlines that expression of mSTIM1 C-Terminal out in breast cancer cells is correlated with the effects on cell survival of anti-STIM1 mAbs targeting the C terminus of STIM1. Amount of mSTIM1 C-Terminal out in the plasma membrane of breast cancer cells was evaluated by an Elisa approach (panel A). Histograms represent the mean values of optical densities (OD) ± SEM measured in intact cells using antibodies targeting the C-terminal region (CDN3H4 clone) of STIM1. Panels B and C: In each experiment, cells were treated for 48 hours with 10 µg/ml of the antibody targeting the C terminal domain of STIM1 (CDN3H4 clone) or with a control isotype. Evaluation of cell proliferation (Cell Titer assay, Panel B) or cell survival (CCK8 kit, Panel C) of two types of breast cancer cell lines treated with the anti-STIM1 antibody (CDN3H4 clone; n=8). Histograms represent for each experimental conditions individual values and the mean +/- SEM of n observations. Data were normalized to the mean values obtained for cells treated with the isotype. Data are analyzed by non parametric Mann Withney analysis, *P<0.05, **P<0.01 and ***P<0.001.

### Examples

### Example 1: Expression of STIM1 protein as a pathology evolution marker of pancreatic adenocarcinoma

We have demonstrated that the expression of STIM1 protein, in cancer cells of patients with pancreatic adenocarcinoma (Pancreatic Ductal AdenoCarcinoma: PDAC) constitutes a pathology evolution marker (see Figure 1).

The expression of STIM1 (average grade) in healthy pancreatic tissues and pancreatic tumor tissues (PDAC) (n=48) is shown in Table 1.

**Table 1:**

| Variable | Average | Standard deviation | Test Student |
|---|---|---|---|
| STIM1 tumor | 1.58 | 0.72 | 0.002** |
| STIM1 healthy | 1.95 | 0.19 | |

| | | | |
|---|---|---|---|
| **P<0.01 | | | |

The expression of STIM1 in healthy pancreatic tissues and cancer pancreatic tissues defined by the percentage of low and high grades (n=48) is shown in Table 2.

**Table 2:**

| Variable | Expression of STIM1 |
|---|---|
| STIM1 tumor | Low: 47.92% |
| | High: 52.08% |
| STIM1 healthy | Low: 8.7% |
| | High: 91.3% |

The expression of STIM1 protein is decreased in cancer cells compared to normal peri-tumoral cells. However the increase in STIM1 expression in cancer cells is correlated with poor prognosis.

STIM1 expression was determined using classical immunohistochemistry protocols. A panel of two different validated antibodies directed against STIM1 was used. Briefly, for IHC analysis of STIM1 expression, formalin-fixed, paraffin-embedded (FFPE) tissue blocks containing both tumor and normal pancreatic tissue were obtained from patients at different pancreatic cancer stages and who had surgery. IHC staining was performed using automated IHC staining system (Roche Diagnostics Ventana Benchmark, N750-BMKU-FS) in accordance with the manufacturer's recommendations. The level of expression of our proteins is scored (1 to 3) corresponding for the score 0 to a weak or nonexistent expression of STIM1 and 3 corresponding to a strong expression of our proteins of interests. Statistical analysis was done using Wilcoxon paired tests.

### Example 2: The level of expression of STIM1 modulates the constitutive entry of calcium

The level of expression of STIM1 modulates the constitutive entry of calcium: (see Figure 2). The amplitude of this entry is related to the level of expression of STIM1 protein at the plasma membrane of pancreatic cancer lines (see Figure 3).

Panc cells were transfected with 5 µg of an expression vector containing the STIM1 gene (pSTIM1 vector) or an empty vector using the transfecting agent lipo293 following commercial recommendations. Cells were transfected with 5 µM of an SiRNA targeting STIM1 (seq: ugagggaagaccucaauua) or a SiCtrl using the transfecting agent lipoRNAimax following commercial recommendations.

For constitutive Ca²⁺ entry (CCE) measurements, Panc cells were loaded with 2 µM of the Fura-2/AM fluorescent dye in the presence of 2 µM Pluronic acid for 60min at 37°C in a medium containing: 135 mM NaCl, 5 mM KCI, 1 mM MgCl₂, 10 mM HEPES, 10 mM Glucose with an 7,4-adjusted pH supplemented with 5 mM CaCl₂. Cells were then washed and displayed on coverslips for single cell fluorescence imaging. Fura-2 was excited alternatively at 340 and 380 nm using a monochromator, and fluorescence emission was recorded at 510 nm using a fluorescence microscope equipped with a dichroic mirror and a 14-bit CCD camera. After the stabilization of basal fluorescence, the extracellular medium was replaced by Buffer A supplemented with 0.5 mM CaCl₂ for 100 s and again with the original 5mM CaCl₂-containing Buffer A after curve stabilization. Values of the ratio of fluorescence measured at 340 and 380 nm are collected over time and normalized.

The amount of mSTIM1 is evaluated by flow cytometry using 5 x 10⁶ cells per condition. Cells were taken off culture plates using an Enzyme free solution for 5 min at 37°C. Cells were then centrifuged for 5 min at 1500 rpm and then incubated with 1 µl of an anti-STIM1 antibody coupled with PE (GOK-PE, BD Biosciences; 20 µg/ml) or with 0,5 µl of an isotype control coupled with PE in 50 µl of PBS on cells for 30 min on ice. After 3 washes, cells were read in PBS using a Flow cytometer (Navios, Beckman coulter).

### Example 3: Demonstration of the double topology of the mSTIM1 protein

We demonstrated by cytometry and Elisa approaches using anti-STIM1 antibodies targeting the C terminal end (clone CDN3H4, sc-66173, Santacruz) and the N terminal end (clone GOK, BD transduction Laboratory) of the STIM1 protein that the fraction of this protein STIM1 localized to the plasma membrane (mSTIM1) has a double topology with an orientation in which the N-terminal domain of the protein is extracellular (called N ter out), as previously established, but also an orientation in which the terminal C domain of mSTIM1 is extracellular (Cter out orientation) (see Figure 4). The dual orientation of mSTIM1 has been demonstrated in pancreatic cells in endogenous expression of STIM1 and using cells over-expressing or under-expressing STIM1 or (see Figures 5 and 6).

Panc cells were transfected with 5 µg of an expression vector containing the STIM1 gene (pSTIM1 vector) or an empty vector using the transfecting agent lipo293 following commercial recommendations. Cells were transfected with 5 µM of an SiRNA targeting STIM1 (sequence: UGAGGGAAGACCUCAAUUA) or a SiCtrl using the transfecting agent lipoRNAimax following commercial recommendations.

The amount of STIM1 was evaluated using flow cytometry or Elisa approaches.

For the ELISA measurements, 10⁶ cells were loaded on 96 wells plates in DMEM medium. Cells were fixed using PFA 2% for 10 min at room temperature (RT). Cells were then washed with Phosphate Buffer Solution (PBS) and next incubated with PBS supplemented with 5% of fat milk for 30 minutes. Cells were next incubated with the anti-STIM1 antibody directed against the N terminus (0,1 µl GOK, BD Biosciences; 1 µg/ml) or the C terminus (10 µl CDN3H4 sc-66173, Santacruz; 1 µg/ml) for 1h30 at RT. After 3 washes with PBS, cells were incubated with in PBS +5%of fat milk containing the peroxidase conjugated secondary antibody for 30 min at RT. After 3 washes, the substrate for peroxidase conjugated secondary antibody (SIGMA*FAST*™ OPD tablets, Sigma-Aldrich) was added for 20 min at 37°C and the reaction was stopped using H₂SO₄ solution. ELISA plate was read at 392 nm in absorbance and the optical density normalized using absorbance emitted at 495 nm by cells following incubation with Janus green.

For determination of the amount of STIM1 using flow cytometry, 5 x 10⁶ cells were used per condition. Pancreatic cells were taken off culture plates using Enzyme free solution for 5 min at 37°C. Cells were then centrifuged for 5 min at 1500 rpm and incubated with 50 µL of PBS containing anti-STIM1 antibody directed against the N terminus (1 µl GOK-PE, BD Biosciences; 20 µg/ml) or the C terminus (5 µl CDN3H4 sc-66173, Santacruz; 10 µg/ml) or 0,5 µL of an isotype control for 30 min on ice. In case of incubation with the uncoupled anti-STIM1, cells were washed and then incubate with a PE conjugated secondary antibody for 20 min on ice. After 3 washes, cells were read in PBS using a Flow cytometer (Navios, Beckman Coulter Life Sciences).

### Example 4: Dual orientation of mSTIM1 in various cancer cell lines

The dual orientation of mSTIM1 has been observed in various pancreatic cell lines (see Figure 7), but also in colon adenocarcinoma cell lines (see figure 21), breast adenocarcinoma cells (see figure 23), B lymphocytes from hematologic malignancies (B-lymphocytes of patients suffering from Chronic lymphocytic leukemia, Burkitt cell lines) (see Figures 8 and 9). Dual topology was identified by flow cytometry or Elisa approaches.

For the ELISA measurements, 10⁶ cells were loaded on 96 wells plates in DMEM medium. Cells were fixed using PFA 2 % for 10 min at room temperature (RT). Cells were then washed with Phosphate Buffer Solution (PBS) and next incubated with PBS supplemented with 5% of fat milk for 30 minutes. Cells were next incubated with the anti-STIM1 antibody directed against the N terminus (0,1 µl GOK, BD Biosciences; 1 µg/ml) or the C terminus (10 µl CDN3H4 sc-66173, Santacruz; 1 µg/ml) for 1h30 at RT. After 3 wash with PBS, cells were incubated in PBS +5% of fat milk containing the peroxidase conjugated secondary antibody for 30 min at RT. After 3 washes, the substrate for peroxidase conjugated secondary antibody (SIGMA*FAST*™ OPD tablets, Sigma-Aldrich) was added for 20 min at 37°C and the reaction was stopped using H₂SO₄ solution. ELISA plate was read at 392 nm in absorbance and the optical density normalized using absorbance emitted at 495 nm by cells following incubation with Janus green.

For determination of the amount of STIM1 using flow cytometry, 5 x 10⁶ cells were used per condition. Pancreatic cells were taken off culture plates using Enzyme free solution for 5 min at 37°C. Cells were then centrifuged for 5 min at 1500 rpm and incubated with 50 uL of PBS containing anti-STIM1 antibody directed against the N terminus (1 µl GOK-PE, BD Biosciences; 20 µg/ml) or the C terminus (5 µl CDN3H4 sc-66173, Santacruz; 10 µg/ml) or 0,5 µL of an isotype control for 30 min on ice. In case of incubation with the uncoupled anti-STIM1, cells were washed and then incubate with a PE conjugated secondary antibody for 20 min on ice. After 3 washes, cells were read in PBS using a Flow cytometer (Navios, Beckman Coulter Life Sciences).

### Example 5: Demonstration of the specificity of the detection of STIM1 by Cter antibodies

By western blot approaches (see Figure 10), it has been shown that an anti-STIM1 antibody targeting the C-terminal domain of the protein (STIM1: clone CDN3H4, sc-66173, Santacruz) well recognizes STIM1 protein. Flow cytometry and Elisa approaches have shown that this antibody recognizes the membrane fraction of STIM1 (mSTIM1). This specificity of the detection of mSTIM1 by Cter antibodies was confirmed by flow cytometry and Elisa by labelling with STIM1 in intact non permeabilized cells expressing different levels of this protein (see Figures 5 and 6). This antibody makes it possible to detect specifically in flow cytometry the membrane fraction of STIM1 (mSTIM1) as well as the majority fraction of STIM1 located at the reticulum membrane endoplasmic on permeabilized cells or broken cells.

Protein extraction was performed by scratching 107 Panc 1-wt cells on ice with a lysis buffer containing: 20 mM Tris HCI pH 7.5, 150 mM NaCl, 1 mM EDTA, 1 mM EGTA, 1% Triton X100, 2.5 mM Na+ pyrosodium tetraphosphate, 1 mM glycerophosphate, 1 mM Na+ orthovanadate, 1 µg/ml leupeptin and a protease inhibitor cocktail. Protein extracts were sonicated and centrifuged for 12 min at 16,000 g. Protein concentration of cell lysates were determined using the Folin method. 75 µg of proteins were run on SDS-PAGE 7,5 % polyacrylamide gels in denaturing conditions, and then transferred onto PVDF (PolyVinyliDene Fluoride) membrane sheets. Unspecific blocking was done by incubation with 5% fat milk in PBS, 0,1 % tween 20 for 1 hour. Blots were incubated overnight with 5% fat milk in PBS, 0.1% tween 20, containing mouse monoclonal anti-STIM1 (CDN3H4 clone Santacruz; 1:1,000 dilution) or mouse monoclonal anti-GAPDH antibody (6C5 clone Abcam; 1:10,000 dilution,). Blots were incubated with Horseradish Peroxydase (HRP)-conjugated goat anti-mouse after washing with PBS, 0,1 % tween 20 and revealed with the Luminata Forte reagent. All results were normalized upon GAPDH quantification.

### Example 6: Constitutive Ca²⁺ entry of pancreatic epithelial cells is inhibited by the use of the anti-STIM1 antibody

We demonstrate that constitutive Ca²⁺ entry of pancreatic epithelial cells is inhibited by the use of the anti-STIM1 antibody clone CDN3H4 (clone CDN3H4, sc-66173, Santacruz) (see Figure 11). This antibody does not have effect on activated Ca²⁺ entry by release of intracellular calcium stores (SOCE: Store Operated Ca²⁺ Entry) (see Figure 12).

For SOCE measurement 10⁶ cells were seeded in 96 wells. Cells are loaded with Fura-2 acetoxymethyl ester (Fura-2 QBT™, Molecular Probes) fluorochrome according to the manufacturer's protocol. The Fura-2 QBT™ was aspirated and replaced by an equal volume of free Ca²⁺ Hepes-buffered solution containing (in mM): 135 NaCl, 5 KCI, 1 MgCl₂, 1 EGTA, 10 Hepes, 10 glucose, pH adjusted at 7.45 with NaOH. Intracellular calcium level variations were monitored by using the FlexStation 3™ (Molecular Devices, Berkshire, UK), Dual excitation wavelength capability permits ratiometric measurements of Fura-2AM peak emissions (510 nm) after excitations at 340 nm (bound to Ca²⁺) and 380 nm (unbound to Ca²⁺). Modifications in the 340/380 ratio reflect changes in intracellular-free Ca²⁺ concentrations. The SOCE was elicited by releasing the Ca²⁺ stores from the endoplasmic reticulum with thapsigargin (2 µM) solution under Ca²⁺-free conditions to determine the magnitude of intracellular Ca²⁺ release (Hepes-buffered solution). Next, cells were returned to a Ca²⁺-containing Hepes-buffered solution to measure SOCE. The magnitude and speed of SOCE were estimated.

For constitutive Ca²⁺ entry (CCE) measurements, Panc-1 cells were loaded with 2 µM of the Fura-2/AM fluorescent dye in the presence of 2 µM Pluronic acid for 60min at 37°C in a medium containing: 135 mM NaCl, 5 mM KCI, 1 mM MgCl₂, 10 mM HEPES, 10 mM Glucose with an 7,4-adjusted pH supplemented with 5 mM CaCl₂. Cells were then washed and displayed on coverslips for single cell fluorescence imaging. Fura-2 was excited alternatively at 340 and 380 nm using a monochromator, and fluorescence emission was recorded at 510 nm using a fluorescence microscope equipped with a dichroic mirror and a 14-bit CCD camera. After the stabilization of basal fluorescence, the extracellular medium was replaced by Buffer A supplemented with 0.5 mM CaCl₂ for 100 s and again with the original 5mM CaCl₂-containing Buffer A after curve stabilization. Values of the ratio of fluorescence measured at 340 and 380 nm are collected over time and normalized.

### Example 7: The presence at the plasma membrane of mSTIM1 having a Cter out orientation is independent of glycosylation of STIM1 and regulated by TGFβ signaling

The presence at the plasma membrane of mSTIM1 having a Cter out orientation is independent of glycosylation of STIM1 (glycosylation at position AA N131 and N171) in contrast to its Nter out orientation (see Figures 13 and 14).

The amount of mSTIM1 having a Cter out orientation is regulated by different cell signalling pathways and especially by the way TGFβ, an over-activated pathway in pancreatic cancer. TGFβ pathway stimulation induces an increased amount of mSTIM1 with Cter orientation in pancreatic cancer cells (see Figure 15).

Panc and Miapaca cells were overexpressed with 5 µg of the expression vector pSTIM1 vector containing wild type STIM1 (pSTIM1) or mutated STIM1 (N131Q N171Q STIM1) or an empty vector using the transfecting reagent lipo293 following commercial recommendations. To evaluate the influence of TGFβ signaling cells were treated with 25 ng/ml TGFβ1 for 5 min, 15 min, 30 min, 24h and 48h in DMEM medium at 37°C.

Amount of STIM1 at the plasma membrane was evaluated by ELISA. 10⁶ cells were loaded on 96 wells plates in DMEM medium. Cells were fixed using PFA 2% for 10 min at room temperature (RT). Cells were then washed with Phosphate Buffer Solution (PBS) and next incubated with PBS supplemented with 5% of fat milk for 30 minutes. Cells were next incubated with the anti-STIM1 antibody directed against the N terminus (1 µl GOK, BD Biosciences; 1µg/ml) or the C terminus (5 µl CDN3H4 sc-66173, Santacruz; 10 µg/ml) for 1h30 at RT. After 3 washes with PBS, cells were incubated with in PBS +5% of fat milk containing the peroxidase conjugated secondary antibody for 30 min at RT. After 3 washes, the substrate for peroxidase conjugated secondary antibody (SIGMA*FAST*™ OPD tablets, Sigma-Aldrich) was added for 20 min at 37°C and the reaction was stopped using H₂SO₄ solution. ELISA plate was read at 392 nm in absorbance and the optical density normalized using absorbance emitted at 495 nm by cells following incubation with Janus green.

### Example 8: Anti-STIM1 antibody targeting the C terminal fraction of STIM1 reduces in vitro migration of Panc-1-Wt pancreatic cancer cells

Anti-STIM1 antibody targeting the C terminal fraction of STIM1 (clone CDN3H4, Santa Cruz) reduces in vitro migration of Panc-1-Wt cells evaluated by transwell migration (Boyden chambers) or by wound healing technique (see Figure 16).

For the migration measurements (wound healing technique), 10⁶ cells were loaded on 96 wells plate in DMEM medium. Panc-1-Wt confluent cells were manually scratched, and cells were treated with anti-STIM1 CDN3H4 (10 ug/ml) or an isotype. Acquisition was done during 48 hours with a rate of one picture per hour. Area of a wound was calculated in images representing the time-series.

For migration assays based on migration through filters, 5 x 10⁶ cells were loaded on the top of a Boyden chamber (transwell Corning 8 µM pores) and incubated with or without anti-STIM1 targeting the C terminus of STIM1 (CDN3H4 clone 10 µg/ml) for 48 hours at 37°C in DMEM media without SVF. The bottom plate was loaded with 10% SVF DMEM medium. After removing cells from the upper face of the filter, five fields of cells were manually counted after fixation of migrated cells and Dapi nucleus coloration.

### Example 9: The anti-STIM1 antibody targeting the C terminal fraction of STIM1 reduces in vitro the survival of Panc-1-Wt cells pancreatic cancer cells, colon cancer cells and breast cancer cells

The anti-STIM1 antibody targeting the C terminal fraction of STIM1 (clone CDN3H4, Santa Cruz) reduces in vitro the survival of pancreatic cancer cells (see Figure 17), colonic pancreatic cancer cells (see Figure 21) and breast cancer cells (see Figure 23). Treatment with Anti-STIM1 antibody targeting the C terminal fraction of STIM1 reduces cell survival mainly by reducing cell proliferation (See Figure 17 and figures 21 and 23). *In vitro,* Anti-STIM1 antibody targeting the C terminal fraction of STIM1 (clone CDN3H4) let to cell cytotoxicity by inducing apoptosis of these cells (see Figure 18 and 22). Different antibody targeting the C terminal end of STIM1 also induces a decrease in cell survival (figure 19). The effects of anti-STIM1 antibodies are proportional to the amount of mSTIM1 orientated with its C terminal end out (figures 21 and 23).

For cell proliferation, cytotoxicity, and cell survival measurements, 10⁶ cells were seeded in 96 wells plates in 100 µL of DMEM medium. Cells were incubated for 48H hours at 37°C with an anti-STIM1 antibody targeting the C terminal of STIM1 (CDN3H4, Santa Cruz ; HPA011088 or HPA012123 clones, Sigma). Cell proliferation was evaluated using the Cell Titer kit proliferation assay (Promega). Cytotoxicty was evaluated using the Cell Tox green cytotoxicity assay (Promega) and cell survival measured using the CCK8 kit from Sigma. All these kits were used following manufacturer recommendations and using a plate reader

For the apoptosis measurements, 5x 10⁶ cells were seeded in 12 wells plates. Cells were incubated for 48 hours with anti-STIM1 CDN3H4 antibody (10 ug/ml) at 37°C. After wash, cells were taken off plates using an enzyme free solution. Cells were centrifuged for 5 min at 1500 rmp and suspended in 50 µL of PBS containing 1 µL of AnnexinV-FiTC and 1 µL of propidium iodure (PI) for 15 min at RT in the dark. Evaluation of Anexin and PI staining was realized using a flow cytometer (Navios, Beckman Counter) to evaluate cell apoptosis and necrosis.

### Example 10 : Association of an anti-STIM1 antibody targeting the C terminal fraction of STIM1 with Gemcitabine potentiates the effect of Gemcitabine on pancreatic cell survival

A low dose (5 µg/ml) of the anti-STIM1 antibody targeting the C terminal fraction of STIM1 (clone CDN3H4, Santa Cruz) or a low dose of Gemcitabine (15 nM) slightly but significantly reduce the survival and proliferation of pancreatic cancer cells (see Figure 17), without inducing cell cytotoxicity. Association of this anti-STIM1 antibody targeting the C terminal fraction of STIM1 with the same low dose of Gemcitabine induces a significant and important potentiation of the effects of a treatment with Gemcitabine alone.

For cell proliferation, cytotoxicity, and cell survival measurements, 10⁶ cells were seeded in 96 wells plates in 100 µL of DMEM medium. Cells were incubated for 48H hours at 37°C with 5 µg/ml of the anti-STIM1 antibody targeting the C terminal of STIM1 (CDN3H4 ,Santa Cruz) alone, with 15 nM Gemcitabine alone or with both 5 ug/ml of the anti-STIM1 clone CDN3H4 and 15 nM Gemcitabine. Relevant controls (isotype or/and DMSO treatments) were also realized. Cell proliferation was evaluated using the Cell Titer kit proliferation assay (Promega). Cytotoxicty was evaluated using the Cell Tox green cytotoxicity assay (Promega) and cell survival measured using the CCK8 kit from Sigma. All these kits were used following manufacturer recommendations and using a plate reader

### Example 11 Method of screening anti-C terminal fragment of mSTIM1

Screening of the molecules modulating the STIM1 fraction localized to the plasma membrane (mSTIM1) with its C terminus out is carried out on cells expressing sufficient amount of mSTIM1 with its C terminus out such as Panc-1 pancreatic cancer cells .Two types of Panc-1 cells are used: Panc-1 cells stably transfected with STIM1 or Panc-1 with endogenous amount of STIM1. These cells display a measurable constitutive calcium entry. Screening consists in a first approach of measuring the effects of the molecules targeting the fraction of STIM1 localized to the plasma membrane of the cells on the constitutive entry of extracellular calcium. The effects of these molecules on calcium entry dependent on the release of reserves SOCE (Store Operated Calcium Entry) are also evaluated in order to determine the effect of the molecules on the influx SOCE of the molecules acting on constitutive calcium entry. The amplitude of these two calcium flows is measured by monitoring the variations in intracellular calcium concentration using a fluorescent probe (Fura-2 QBT, Molecular Devices).

For constitutive Ca²⁺ entry (CCE) measurements, Panc cells were loaded with 2 µM of the Fura-2 QBT fluorescent dye for 60min at 37°C in a medium containing: 135 mM NaCl, 5 mM KCI, 1 mM MgCl₂, 10 mM HEPES, 10 mM Glucose with an 7,4-adjusted pH supplemented with 1,8 mM CaCl₂. Cells were then washed and displayed on coverslips for single cell fluorescence imaging. Fura-2 was excited alternatively at 340 and 380 nm using a monochromator, and fluorescence emission was recorded at 510 nm using a fluorescence microscope equipped with a dichroic mirror and a 14-bit CCD camera. After the stabilization of basal fluorescence, the extracellular medium was replaced by Buffer A supplemented with 0.5 mM CaCl₂ for 100 s and again with the original 5mM CaCl₂-containing Buffer A after curve stabilization. Values of the ratio of fluorescence measured at 340 and 380 nm are collected over time and normalized.

For SOCE and Constitutive Ca2+ entry measurements, 10⁶ cells were seeded in 96 wells. Cells are loaded with Fura-2 acetoxymethyl ester (Fura-2 QBT™, Molecular Probes) fluorochrome according to the manufacturer's protocol. The Fura-2 QBT™ was aspirated and replaced by an equal volume of free Ca²⁺ Hepes-buffered solution containing (in mM): 135 NaCl, 5 KCI, 1 MgCl₂, 1 EGTA, 10 Hepes, 10 glucose, pH adjusted at 7.45 with NaOH. Changes in fluorescence are monitored by using the FlexStation 3™ (Molecular Devices, Berkshire, UK). Dual excitation wavelength capability permits ratiometric measurements of Fura-2AM peak emissions (510 nm) after excitations at 340 nm (bound to Ca²⁺) and 380 nm (unbound to Ca²⁺). Modifications in the 340/380 ratio reflect changes in intracellular-free Ca²⁺ concentrations. The SOCE was elicited by releasing the Ca²⁺ stores from the endoplasmic reticulum with thapsigargin (2 µM) solution under Ca²⁺-free conditions to determine the magnitude of intracellular Ca²⁺ release (Hepes-buffered solution). Next, cells were returned to a Ca²⁺-containing Hepes-buffered solution to measure SOCE. The magnitude and speed of SOCE were estimated. Constitutive entry is measured in the absence of any stimulation by following the quench of Fura 2 fluorescence recorded at 360 nm wavelength excitation when manganese (Mn²⁺) is added to the extracellular medium. The rate of fluorescence quenching represents a good approximation of Ca²⁺ entry.

The cells are put in contact with the test compound at the moment of loading the cells with the fluorescent probe and throughout measurement of the variations in intracellular calcium concentration.

The effects of screened compounds on STIM1 location at the plasma membrane is realized for lead candidates. Screening is also done by following the effects of promising molecules on cell migration and cell survival.

### Reference List

1. Mignen O, Thompson JL, Shuttleworth TJ. STIM1 regulates Ca2+ entry via arachidonate-regulated Ca2+-selective (ARC) channels without store depletion or translocation to the plasma membrane. J Physiol. (2007) 579:703-15.
2. Remington Pharmaceutical Sciences (Mack Publishing Company, Easton, USA, 1985.

## Claims

1. Substance interacting with C terminal fragment of the STIM1 fraction localized to the plasma membrane of the cells, for its use in the treatment of cancers.

2. Substance for use according to claim 1, wherein said cancer is a cancer with cells having a STIM1 fraction localized to the plasma membrane of the cells and with at least a part of its C terminal end being extracellular.

3. Substance for use according to claim 1 or 2, wherein said cancer is for example pancreatic cancer, colon cancer, breast cancer, Burkitt lymphoma or chronic Lymphocytic Leukemia.

4. Substance for use according to claim 3, wherein said pancreatic cancer is pancreatic ductal adenocarcinoma.

5. Substance for use according to anyone of the preceding claims, wherein said substance is chosen among an antibody or a fragment thereof, a protein, a peptide, a chemical compound and an aptamer.

6. Substance for use according to anyone of the preceding claims, wherein said substance is in combination with at least one other active ingredient, such as gemcitabine.

7. Pharmaceutical composition comprising at least one substance interacting with C terminal fragment of the STIM1 fraction localized to the plasma membrane of the cells, and at least one pharmaceutically acceptable excipient.

8. Pharmaceutical composition according to claim 7, further comprising at least one other active ingredient, such as gemcitabine.

9. Use of isolated C terminal fragment of the STIM1 fraction localized to the plasma membrane of the cells in a method for screening candidate molecules for treating cancers, especially pancreatic, colon, breast cancer, Burkitt lymphoma or chronic Lymphocytic Leukaemia .

10. Use according to claim 9, wherein the method of screening uses a technique selected from the group comprising biological screening, and biophysical screening.

11. Use according to claim 10, wherein screening uses a technique selected from the group comprising immunofluorescence, Western blot, immunoprecipitation, surface plasmon resonance (SPR), flow cytometry, video microscopy, study of calcium flows, enzyme-linked immunosorbent assay (ELISA), fluorescence and luminescence complementation assays and confocal microscopy.

12. Process for predicting the progression and/or monitoring the progression of a cancer, comprising the in vitro detection of the expression of STIM1 in a sample of the tumour of the subject.

13. Process according to claim 12, wherein said cancer is pancreatic cancer.

14. Process according to anyone of claims 12 or 13, wherein said detection is made with an antibody directed against the C terminal fragment of the STIM1 fraction located at the cell plasma membrane.
